(19)

Europäisches
Patentamt
European
Patent Office
Office européen
des brevets

(11) **EP 2 138 587 A1**

(12) **EUROPEAN PATENT APPLICATION**

(43) Date of publication:
**30.12.2009 Bulletin 2009/53**

(51) Int Cl.:
***C12Q 1/68*** *(2006.01)*

(21) Application number: **08158774.3**

(22) Date of filing: **23.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Koninklijke Philips Electronics N.V.
5621 BA Eindhoven (NL)**

(72) Inventor: **The designation of the inventor has not yet been filed**

(74) Representative: **Damen, Daniel Martijn
Philips
Intellectual Property & Standards
P.O. Box 220
5600 AE Eindhoven (NL)**

(54) **Amplification of nucleic acids using temperature zones**

(57) The present invention relates to the amplification and detection of amplified nucleic acid sequences employing methods and devices with distinct temperature zones. The methods and devices of the present invention may be used for quantitative analysis of target nucleic acid sequences, for simultaneous quantitative analysis of multiple target nucleic acid sequences or for analyzing a sample for the presence of a target nucleic acid.

Fig 10:

EP 2 138 587 A1

**Description**

FIELD OF THE INVENTION

The present invention relates to a process for conducting amplification of nucleic acid sequences and to a device for conducting the method of the present invention. The invention is especially suited for the simultaneous identification and quantification of nucleic acids present in a sample, e.g. a biological sample. Further, the present invention relates to a device for amplification and quantitative real-time detection of target nucleic acid sequences in an amplification reaction, particularly in a polymerase chain reaction (PCR) in an amplification solution in a reaction container comprising at least two kind of zones that are thermally decoupled from each other, wherein one zone is a surface hybridization zone having a substantially constant generated temperature allowing for hybridization of oligonucleotide probes to nucleic acid sequences, wherein an inner surface of the surface hybridization zone comprises capture probes for target nucleic acid sequences.

BACKGROUND OF THE INVENTION

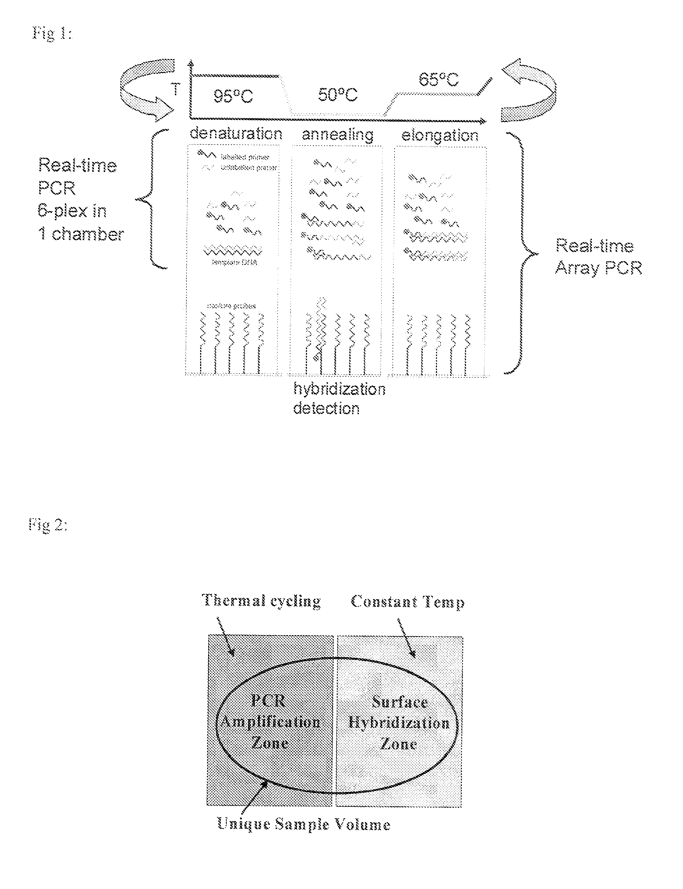

Amplification of nucleic acid sequences can be performed using a variety of amplification reactions, among them and most prominent the polymerase chain reaction (PCR). PCR is a method for amplification of specific nucleic acid sequences. PCR is an enzymatic reaction that primarily takes place in solution. When the amplification process is monitored in real-time, PCR can be used for quantitative analysis. Real-time PCR normally uses fluorescent reporters, such as intercalating dyes, TaqMan probes, Scorpion primers, molecular beacons. When more than one nucleic acid target sequence is to be analyzed, two approaches can be taken. The first approach is to parallelize the reactions, i.e. to run each reaction in a separate compartment. The second approach is to multiplex the reactions, i.e. to run the reactions in the same compartment and to use different fluorophore reporters for each reaction. This approach is limited by the number of fluorophores that can efficiently be discriminated. The current state-of-the-art is that six reactions can be multiplexed. Appended figure 1 illustrates current single-chamber multiplex-PCR and array PCR concepts.

The thermal requirements of the PCR process and of the surface hybridization process are very different. The PCR amplification process requires (fast) temperature cycling wherein part of the cycle the sample is above the dsDNA melting temperature. On the other hand the surface hybridization needs to take place at a temperature below the nucleic acid melting point, and for optimum performances diffusion limited local depletion of amplicons above the capture probe sites should be avoided.

The present multiple-zone concept allows decoupled optimization of these two processes, i.e. the amplification and the surface hybridization.

SUMMARY OF THE INVENTION

The present invention relates to a method and device for the amplification and detection of target nucleic acid sequences using distinct temperature zones. In particular, the present invention relates to a method for amplification and detection of target nucleic acid sequences in an amplification solution (which possibly contains nucleic acids with the target nucleic acid sequences) in a reaction container, comprising the steps of (a) providing a reaction container comprising at least one surface hybridization zone in which capture probes are immobilized on a surface, wherein said capture probes are substantially complementary to regions on said target nucleic acid sequences; (b) adding the amplification solution to said reaction container; (c) generating a temperature zone profile in the reaction container with at least two kinds of thermally decoupled zones, wherein one kind of zone is identical or at least overlapping to/with the surface hybridization zones, wherein the surface hybridization zones have a generated temperature allowing for hybridization of the capture probes to the target nucleic acid sequences; (d) performing an amplification of target nucleic acid sequences in the reaction container; and (e) detecting amplified nucleic acid sequences in periodic or defined intervals during and/or after amplification, wherein amplified nucleic acid sequences are detected by hybridization of capture probes to said amplified nucleic acid sequences in said surface hybridization zone. In a particularly preferred embodiments of the method either (a) each surface hybridization zone is used for hybridization and detection of amplified target nucleic acid sequences only at a particular stage of the entire amplification process, or (b) at least one surface hybridization zone is used for hybridization and detection of amplified target nucleic acid sequences at multiple stages of the entire amplification process. Preferably, the amplification is performed in a polymerase chain reaction (PCR) and the amplification solution comprises a nucleic acid polymerase and primers substantially complementary to said target nucleic acid.

The present invention also relates to a device for amplification and detection of target nucleic acid sequences in an amplification solution in a reaction container, comprising (a) a reaction container comprising at least two kind of zones that are thermally decoupled from each other, wherein one kind of zone is a surface hybridization zone having a substantially constant generated temperature allowing for hybridization of capture probes to complementary target nucleic acid sequences, wherein an inner surface of the surface hybridization zone is coated with capture probes for target nucleic acid sequences, (b) a detection system that detects targets which are bound to said capture probes, (c) one or more temperature controllers and one or more temperature adjusters for controlling, adjusting and maintaining the temperature in the zones, and (d) a transportation system for transporting the amplification solution between the zones. Preferably, the detection system does essentially not detect targets which are not bound to said capture probes.

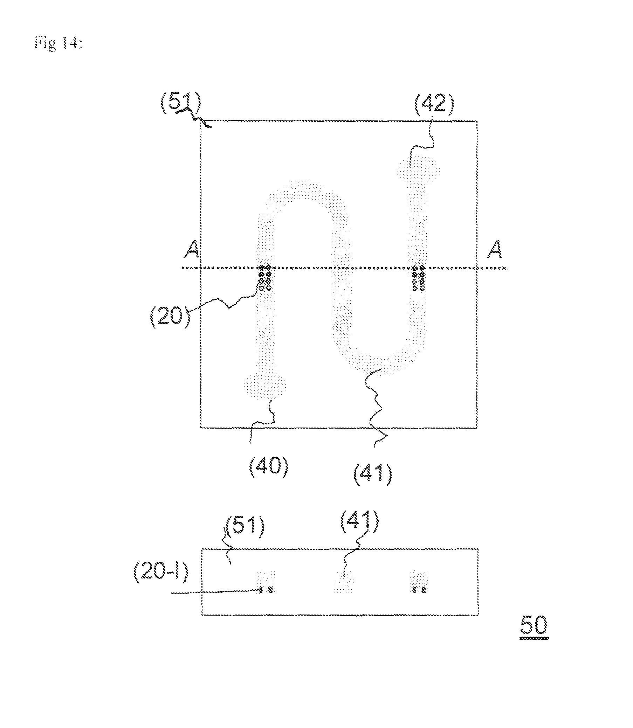

The present invention also relates to a cartridge for amplification and detection of target nucleic acid sequences in an amplification solution in a reaction container, comprising a reaction container for receiving an amplification solution comprising said target nucleic acid sequences, wherein the reaction container comprises at least one surface hybridization zone in which capture probes are immobilized on a surface, wherein said capture probes are substantially complementary to regions on said target nucleic acid sequences. Preferably, the reaction container comprises at least two surface hybridization zones in which capture probes are immobilized on a surface, wherein said capture probes are substantially complementary to regions on said target nucleic acid sequences. The features of some illustrative embodiments of the cartridge have already been discussed herein above in context of the cartridge as a component of the device of the present invention. A particular embodiment of such a cartridge is illustrated in appended figure 14. When inserted into the device the surface hybridization zone in the cartridge preferably overlaps with the zone of the device in which a temperature allowing for hybridization is generated.

Thus, the present invention also relates to a device for receiving the cartridge of the present invention, wherein the device comprises:

one or more temperature controllers and/or temperature adjusters for generating a temperature profile of at least two kind of temperature zones in a reaction container comprised in said cartridge, wherein one kind of zone has a substantially constant generated temperature allowing for hybridization of capture probes to complementary target nucleic acid sequences and wherein the temperature controllers and/or temperature adjusters control, adjust and maintain the temperature in the zones;

a detection system that detects targets which are bound to said capture probes but does essentially not detect targets which are not bound to said capture probes;

a transportation system for transporting the amplification solution between the zones; and

a receiving element for said cartridge.

It is preferred that when a cartridge is inserted into the device the surface hybridization zone of the cartridge overlaps with the zone of the device in which a temperature allowing for hybridization is generated.

It is preferred that the reaction container is comprised in an exchangeable cartridge.

Also within the scope of the present invention is the use of the methods, cartridges and devices of the present invention for quantitative analysis of target nucleic acid sequences, for simultaneous quantitative analysis of multiple target nucleic acid sequences or for analyzing a sample for the presence of a target nucleic acid. Preferably, the methods and devices described herein are used for clinical diagnosis, point-of care diagnosis, bio-molecular diagnostics, gene or protein expression arrays, environmental sensors, food quality sensors or forensic applications. The present invention also relates to the use of the methods, cartridges and devices of the present invention in real-time PCR or real-time multiplex PCR.

BRIEF DESCRIPTION OF THE DRAWINGS

Fig. 1: Illustration of steps during real-time array PCR, which comprises 3 temperature steps per cycle, similar to real-time PCR. The difference between real-time array PCR illustrated in the illustrative example of Fig. 1 and real-time PCR in general is that in real-time array PCR the annealing step is combined with a hybridization step.

Fig. 2: Illustration of the zone concept with thermally isolated zones in a unique sample volume.

Fig. 3: Possible embodiment of the zone concept with separate chambers. Ideally, but not limited to, the temperature of the surface hybridization zone is close to the temperature of the annealing temperature of the PCR cycle.

Fig. 4: Possible embodiment of the zone concept with separate chambers, where the sample can be transported around the chambers. Ideally, but not limited to, the temperature of the surface hybridization zone is close to the temperature of the annealing temperature of the PCR cycle.

Fig. 5: Possible embodiment with three constant temperature zones, with the sample circulated among them.

Fig. 6: Possible embodiment with two constant temperature zones, with the sample circulated between them. Preferred embodiment for short length amplicons.

Fig. 7: Two constant temperature zones with the sample circulated around. One zone has the form of a channel with capture probe sites for surface hybridization.

Fig. 8: Supporting experimental evidence of fast surface hybridization of PCR product.

Fig. 9: Correlation between the amplicons concentrations and the hybridization signal.

Fig. 10: Schematic layout of an embodiment according to the invention. With: (1,2,3) Temperature zones for denaturation (1), hybridization and primer annealing (2), and elongation (3). (10) Meandering flow (channel) such that for each cycle (N, N+1, N+2, ...)

the PCR buffer passes the temperature zones; where the meander runs from the left to the right. (20) Dedicated hybridization zone for each cycle (in principle one can skip the hybridization for a cycle). (21) Capture probe sites.

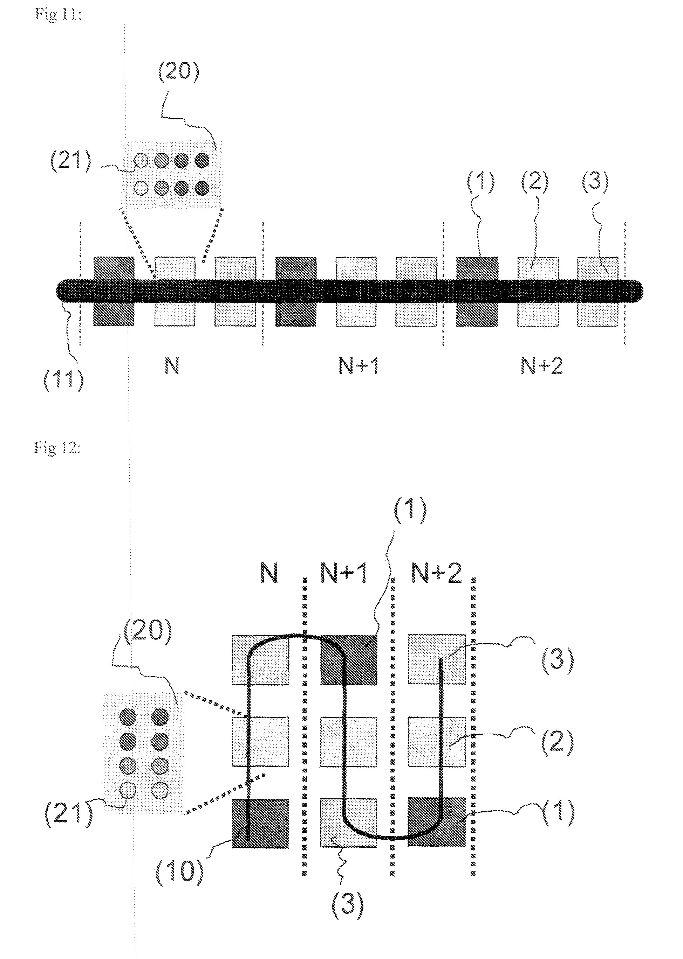

Fig. 11: Schematic layout of another embodiment according to the invention. With: (1,2,3) Individually addressable temperature zones for denaturation (1), hybridization and primer annealing (2), and elongation (3) for cycle (N+2). (11) Unidirectional, non-circulating flow (channel) such that for each cycle (N, N+1, N+2, ...) the PCR fluid passes the individually addressable temperature zones for that particular cycle; where the flow runs from the left to the right. (20) Dedicated hybridization zone for each cycle (in principle one can skip the hybridization for a cycle). (21) Capture probe sites.

Fig. 12: Schematic layout of a preferred alternative embodiment using a meandering flow (10) in order to have the distance between hybridization zones of the PCR cycles as small as possible, which is advantageous from a detection point of view; the larger the distance between the hybridization zones for adjacent PCR cycles the faster scanning (in case of a scanning optical reader) or the larger field of view (in case of an imaging optical reader) is needed.

Fig. 13: 2D matrix of temperature zones (p,q). The settings of the temperature zones are in conformity with the shape of the fluid channels (12), (13) of the cartridge.

Fig. 14: Top view and cross sectional view (along A-A) of a cartridge according to the invention, with:

(50) Cartridge

(51) Body housing of the cartridge, which comprises an at least partly transparent material to enable optical detection. The cartridge is preferably made of (but is not limited to) plastic and like materials or glass. The cartridge can also be an assembly of a transparent substrate with capture probes and a box containing fluid channels.

(20) Sites (typically spots) with capture probes that are substantially complementary to target nucleic acid sequences. Typically, but by no means limiting, the capture probe sites are spots 0.1 mm in diameter. (20-I) a capture probe site that is also visible in the cross-sectional view along line A-A.

(40) Inlet for fluid.

(41) Fluid channel with a meandering layout for this particular example, typical dimensions of the fluid channel are a height of 0.05-1 mm and a width of 0.1-1 mm.

(42) Outlet for fluid

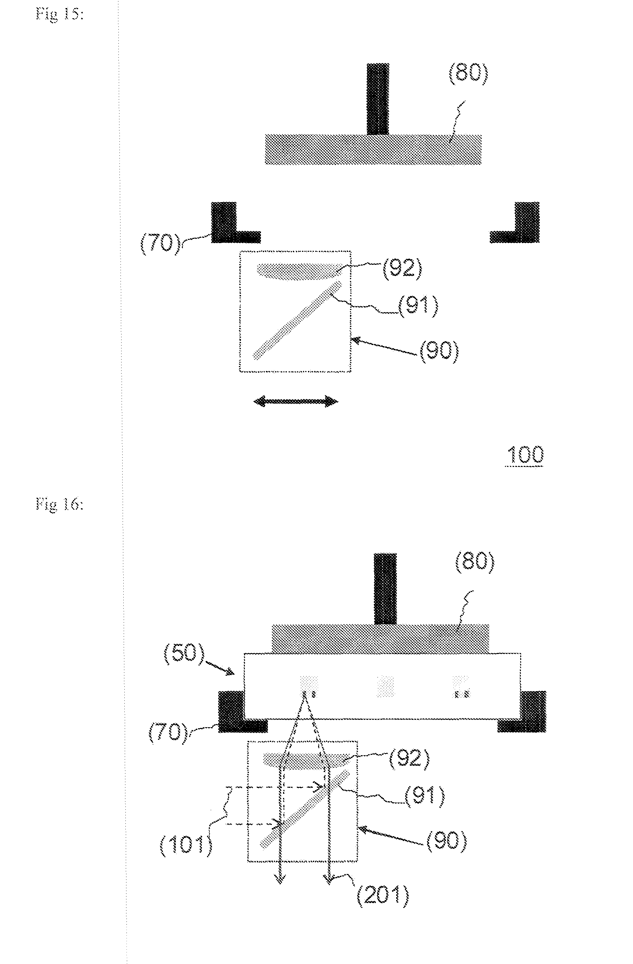

Fig. 15: Device (100) according to the present invention without the cartridge comprising the reaction chamber. The device is ready to receive a (disposable) cartridge with the reaction chamber. The device comprises:

(70) Sample holder for cartridge (50)

(80) Heaters for defining the temperature in the zones (1)-(3) of the cartridge, for this particular case there will be three heaters (80) to define the temperatures in zones (1), (2), (3).

(90) Detection system ("reader") for detecting the target nucleic acids that are hybridized to the capture probes (20). A simplified schematic illustration is shown of a scanning unit for confocal detection. Where the scanning unit comprises an illumination/collection (of the fluorescence) lens/objective (92) and a dichroic mirror (91) that reflects the excitation light (101 in Fig. 16) resulting in excitation light directed towards the cartridge. The same lens (92) collects the fluorescence (201 in Fig. 16) generated by the labeled target molecule and directs this fluorescence via the dichroic mirror (91), which transmits the fluorescent light, towards a detector (not shown here).

Fig. 16: Device of Fig. 15 with cartridge (50) comprising the reaction chamber. The numbering is identical to the numbering of Fig. 15.

DETAILED DESCRIPTION OF EMBODIMENTS

The present invention relates to a method for amplification and detection of target nucleic acid sequences in an amplification solution in a reaction container, comprising the steps of

providing a reaction container comprising at least one surface hybridization zone in which capture probes are immobilized on a surface, wherein said capture probes are substantially complementary to regions on said target nucleic acid sequences;

adding the amplification solution to said reaction container;

generating a temperature zone profile in the reaction container with at least two kinds of thermally decoupled zones, wherein one kind of zone is identical or at least overlapping to/with the surface hybridization zones, wherein the surface hybridization zones have a generated temperature allowing for hybridization of the capture probes to the target nucleic acid sequences;

performing an amplification of target nucleic acid sequences in the reaction container; and

detecting amplified nucleic acid sequences in periodic or defined intervals during and/or after amplification, wherein amplified nucleic acid sequences are detected by hybridization of capture probes to said amplified nucleic acid sequences in said surface hybridization zone .

"Generating a temperature zone profile" herein means that a local or spatial temperature pattern or profile of different zones is induced within the reaction container by temperature controllers and/or adjusters (e.g. comprising heaters, coolers, heat transfer means and the like). A zone or temperature zone herein relates to a defined area or volume within the reaction container. The zones may preferably be thermally decoupled zones. The zones are preferably defined by their temperature and a temperature zone herein relates to a zone of defined temperature, which might be a substantially constant generated temperature or a temperature which varies during the course of the methods of the invention, however, this depends on the type of zone and on the particular embodiment as discussed herein below.

In a further aspect the present invention relates to a method for amplification and detection of target nucleic acid sequences in an amplification solution in a reaction container, comprising the steps of

providing a reaction container comprising said amplification solution and further comprising at least two kinds of thermally decoupled zones, wherein one kind of zone is a surface hybridization zone;

performing an amplification of target nucleic acid sequences in the reaction container; and

detecting amplified nucleic acid sequences in periodic or defined intervals during and/or after amplification, wherein amplified nucleic acid sequences are detected by hybridization of capture probes to said amplified nucleic acid sequences and wherein said hybridization takes place in said surface hybridization zone and said capture probes are immobilized on a surface and are substantially complementary to regions on said target nucleic acid sequences.

In some embodiments of the methods of the present invention amplified nucleic acid sequences are detected in periodic or defined intervals during amplification.

The "reaction container" in the context of the present invention is the sum of any containers comprising the reaction solution. The reaction container may in the different embodiments of the invention comprise different numbers of zones and compartments and may have different forms and dimensions as exemplarily discussed herein for the various embodiments. The term "reaction container" is not limited to reaction tubes, capillaries, cuvettes, well plates and the like but also comprises more complex arrangements according to the particular embodiments with two or more compartments and/or systems of interconnected tubes, compartments, channels and/or capillaries and the like. The reaction container is a container comprising at least one compartment for containing an amplification solution. Optionally, the reaction container comprises additionally fluidic elements such as fluid channels for transporting the amplification fluid between the said compartments or pumps for defining a flow through the said fluid channels.

In a preferred embodiment of the method, said amplification solution is passed through said at least two thermally decoupled zones during amplification and detection.

Nucleic-acid amplification in the context of the present invention may be accomplished by any of the various nucleic-acid amplification methods known in the art, including but not limited to the polymerase chain reaction (PCR), ligase chain reaction (LCR), transcription-based amplification system (TAS), and Qβ amplification. Also the application of variants of these methods, e.g. reverse transcription PCR, real-time PCR, asymmetric PCR, or hot-start PCR may be performed according to the methods of the present invention or in the devices according to the present invention.

Preferably, the amplification is performed in a polymerase chain reaction (PCR) and the amplification solution comprises a nucleic acid polymerase and primers substantially complementary to said target nucleic acid.

In a particularly preferred embodiments of the method either

each surface hybridization zone is used for hybridization and detection of amplified target nucleic acid sequences only at a particular stage of the entire amplification process, or

at least one surface hybridization zone is used for hybridization and detection of amplified target nucleic acid sequences at multiple stages of the entire amplification process.

A "particular stage" herein can for example mean during and/or after a particular cycle of an amplification reaction, e.g. a particular PCR cycle. In other words, multiple amplification cycles may in these embodiments share a common surface hybridization zone.

In a particular embodiment, only one surface hybridization zone for the detection of the amplified target nucleic acid is present in the reaction container. In this embodiment, for each PCR cycle or for each PCR cycle in which detection of the amplified target nucleic acid is desired, the detection occurs in the same surface hybridization zone, i.e. only one surface hybridization zone is present.

"Multiple stages" may in a further embodiment for example relate to the fact that a hybridization zone is used for hybridization and detection during and/or after each amplification cycle.

In a particular embodiment, for every detection step a dedicated surface hybridization zone is present in the reaction container. I.e., for each amplification cycle or for each amplification cycle in which detection of the amplified target nucleic acid is desired, the detection occurs in a different surface hybridization zone, i.e. the number of surface hybridization zones represents the number of detection steps, or in a very particular embodiment the number of amplification cycles when detection occurs during or after every cycle of the polymerase chain reaction. This means that for at least one amplification cycle a dedicated surface hybridization zone is present.

According to a particular embodiment, preferably at least one hybridization zone is used for hybridization and detection at multiple stages of the entire amplification process, and two or more of the at least two thermally decoupled zones are comprised in the same compartment or volume of the reaction container.

Preferably in such an embodiment, the aspect ratio of the dimensions of the reaction container is such that at least two thermally decoupled zones can be maintained in one compartment or volume, e.g. two neighboring zones are separated by a suitable distance in at least one dimension. For example, a compartment of a reaction container may in particular embodiments have dimensions in the millimeter/centimeter range and in one or two other dimension in the micrometer range (leading to a high aspect ratio between the dimensions) and the two neighboring zones are separated from each other by several millimeters or centimeters.

In yet another embodiment it is preferred that at least one hybridization zone is used for hybridization and detection at multiple stages of the entire amplification process, and the at least two thermally decoupled zones are comprised in separate compartments or volumes of the reaction container.

Preferably, the reaction container comprises at least one surface hybridization zone with substantially constant generated temperature and at least one thermocycler zone with variable temperature in the range of from the melting point (melting or freezing temperature) to the boiling point (boiling temperature) of the amplification solution (preferably within the range of from about 0°C to 100°C), wherein the amplification solution is transferred from the thermocycler zone to the surface hybridization zone for detection of the amplified target nucleic acid and vice versa for further amplification.

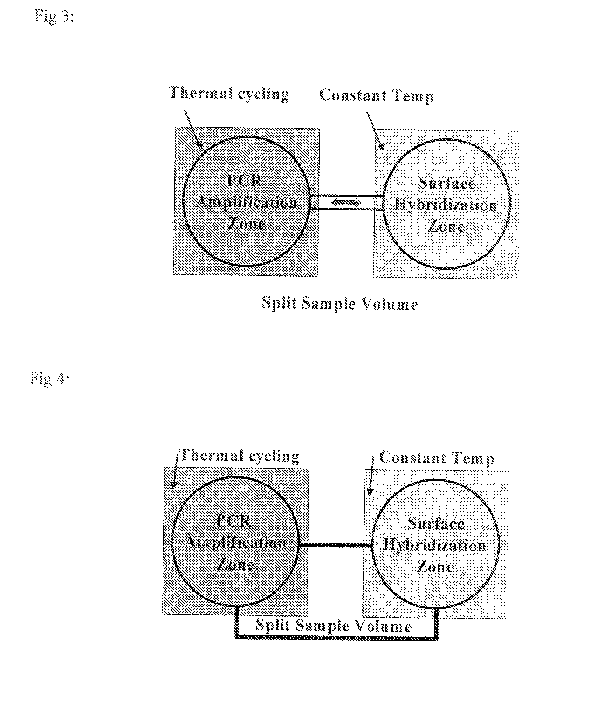

The terms "thermocycler zone" and "PCR amplification zone" are used synonymously herein. Particular layouts of embodiments of the methods according to the present invention comprising a thermocycler zone are illustrated in appended figures 2 to 4, wherein figure 2 illustrates an embodiment, where two zones share the same volume and figures 3 and 4 demonstrate embodiments with split volume, i.e. separate compartments for the hybridization zone and the thermocycler zone.

In a preferred embodiment of the reaction container may preferably comprise at least one thermocycler zone and at least one surface hybridization zone and the amplification reaction is a polymerase chain reaction (PCR) and at least denaturation and primer extension of the polymerase chain reaction are performed in the thermocycler zone and the temperature in the thermocycler zone is cycled at least between denaturation temperature and extension temperature.

It is preferred that primer annealing to said target nucleic acid is performed in the thermocycler zone and the temperature in the thermocycler zone is cycled between denaturation temperature, annealing temperature and extension temperature.

Alternatively, it is also preferred that the temperature in the surface hybridization zone is suitable for primer annealing to said target nucleic acid and that primer annealing to said target nucleic acid is performed in the surface hybridization zone.

The amplification solution may for example be transferred bidirectional or unidirectional and circular between the thermocycler zone(s) and the surface hybridization zone(s) and vice versa.

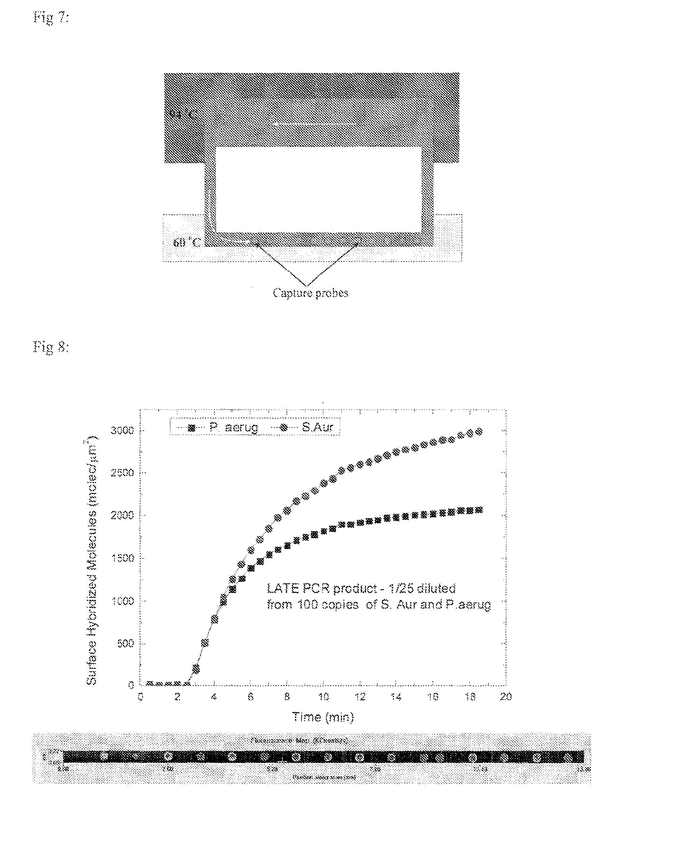

Thus, in some embodiments, the amplification solution may be transferred between the thermocycler zone and the surface hybridization zone in a circular manner (unidirectional) (Fig. 4 and Fig. 7) or in a back-and-forth manner (bidirectional) (Fig. 3). In the case where two or more zones share a volume/compartment, the transfer between the zones may occur by means of convection.

In a particular embodiment of the method, the reaction container comprises at least one surface hybridization zone with substantially constant generated temperature and at least one further zone with substantially constant generated temperature, wherein the temperature of said further zone is adjustable in the range of from the melting point (melting or freezing temperature) to the boiling point (boiling temperature) of the amplification solution (preferably within the range of from about 0°C to 100°C) and wherein the amplification solution is transferred for detection between the surface hybridization zone and the further zone and vice versa.

Preferably, the further zone is a denaturation zone and the temperature of the denaturation zone is suitable for denaturation of the target nucleic acid or may be adjusted to said temperature.

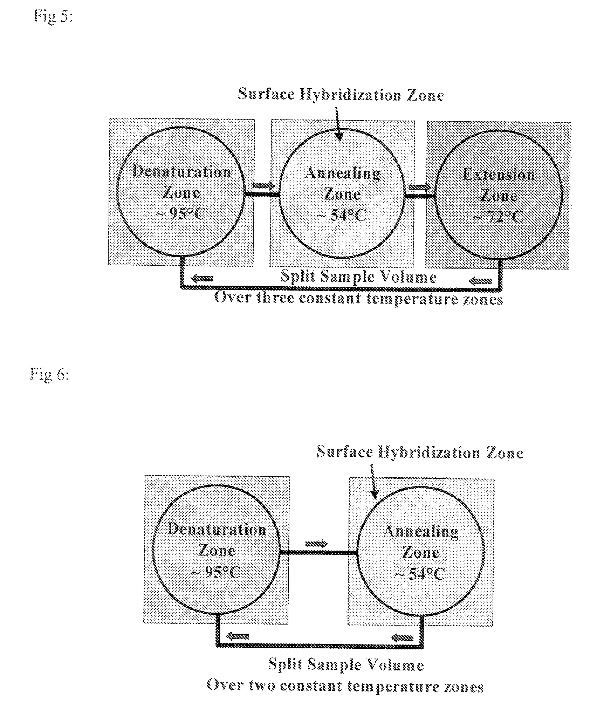

Such an embodiment is for example illustrated in appended figures 6 and 7.

Optionally, the reaction container further comprises an extension zone having a substantially constant generated temperature suitable for primer extension, wherein the target nucleic acid serves as a template and wherein primer annealing is performed in the surface hybridization zone and wherein the amplification solution is cycled through all zones such that polymerase chain reaction and detection may occur.

Such an embodiment is for example illustrated in appended figure 5.

Preferably, the reaction container further comprises an extension zone having a substantially constant generated temperature suitable for primer extension and an annealing zone having a substantially constant generated temperature suitable for primer annealing, wherein primer annealing is performed in the annealing zone and wherein the amplification solution is cycled through all zones such that polymerase chain reaction and detection may occur.

In another embodiment the present invention relates to a method for amplification and detection of target nucleic acid sequences, wherein each surface hybridization zone is used for hybridization and detection of amplified target nucleic acid sequences only at a particular stage of the entire amplification process, and wherein in the reaction container two or more kinds of thermally decoupled zones are generated,

wherein each zone has a substantially constant generated temperature, and

wherein the first kind of zone is a surface hybridization zone and the second and further kind is an amplification zone,

wherein the amplification solution is passed through all zones such that for each amplification cycle at least one amplification zone is passed through and for each amplification cycle in which hybridization and detection is desired additionally a surface hybridization zone is passed through,

and wherein the transport is unidirectional and non-circular.

An amplification zone in this context is a zone in which at least one of the steps for amplification of nucleic acid sequences may occur. This may for example be a denaturation zone or an extension zone or an annealing zone. However, if PCR is used for amplification, for each cycle in such an embodiment a denaturation zone and an extension zone and an annealing zone are present. In some embodiments the annealing zone may be identical to the surface hybridization zone. The sequence in which the zones are passed through resembles the phases of an amplification reaction, e.g. the phases of PCR cycles.

In a preferred embodiment the reaction container comprises three or more kinds of thermally decoupled zones, wherein each zone has a substantially constant generated temperature, and wherein the first kind of zone is a surface hybridization zone, the second kind is a denaturation zone and the third kind is an extension zone, wherein for each amplification cycle one surface hybridization zone is present and the number of surface hybridization zones is equal to the number of denaturation zones and extension zones,

wherein the amplification solution is passed through all zones such that for each amplification cycle first a denaturation zone, secondly a surface hybridization zone and thirdly an extension zone is passed, wherein the transport is unidirectional and non-circular, and wherein the denaturation zone has a temperature allowing for denaturation of the target nucleic acid sequences, the surface hybridization zone has a temperature allowing for annealing of primers and hybridization of oligonucleotide probes (i.e. capture probes) and the extension zone has a temperature allowing for primer extension.

In a very particular embodiment of this particular method, a fourth kind of zone may be present ("annealing zone"), such that primer annealing and surface hybridization occur in different kind of zones. In this embodiment, the temperature in the surface hybridization zone may or may not be identical to the temperature of the annealing zone, i.e. the temperature of the surface hybridization zone may or may not allow for primer annealing.

In a particular embodiment, an amplification solution is transported through the totality of zones by for example a flow and the part of the amplification solution in a particular zone resembles a particular stage in the amplification process. The flow may for example in some embodiments be a constant flow or may in other embodiments be stepwise.

In one embodiment the temperature in all zones of one kind is equal and adjusted concertedly or wherein the temperature in all zones is adjusted separately.

In some embodiments of the present invention, the amplification solution may be passed through additional zones, e.g. before the first denaturation zone or after the last extension zone or after the last surface hybridization zone.

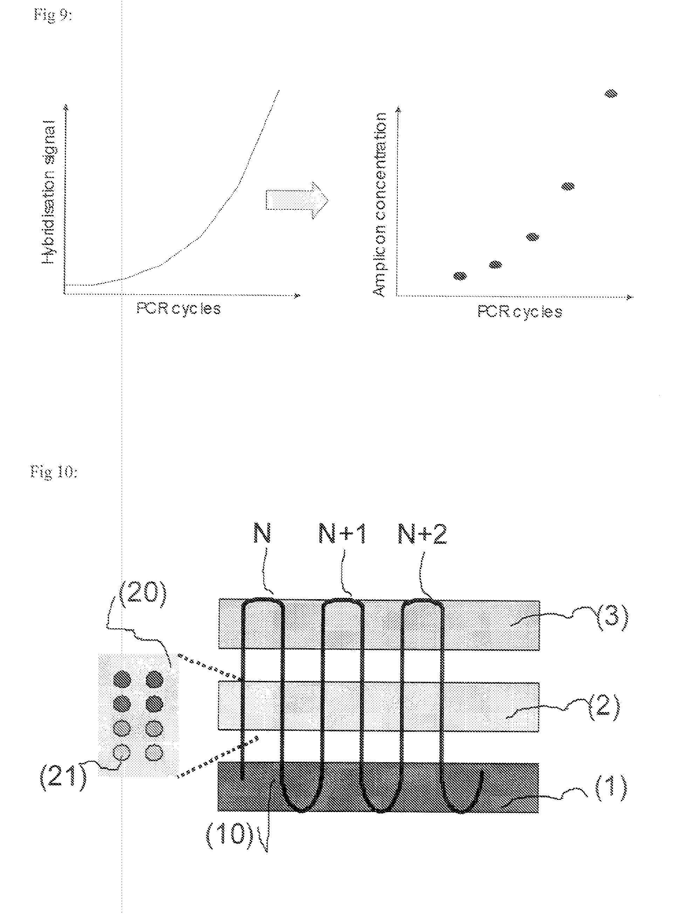

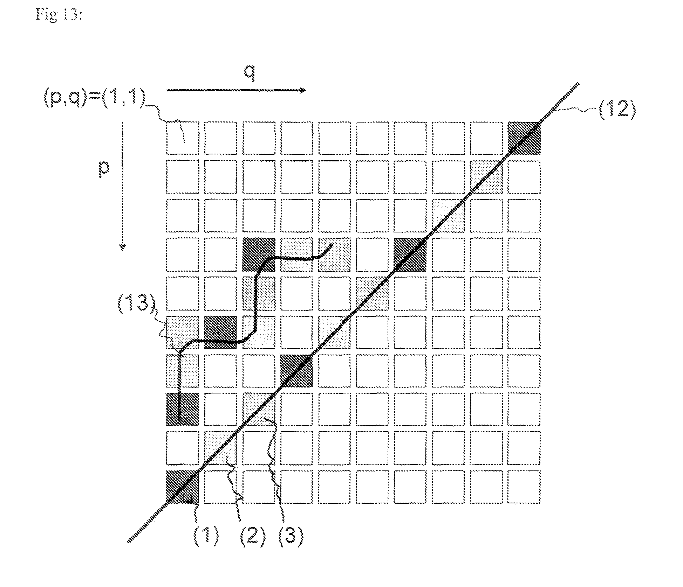

In particular embodiments of the present invention the zones through which the amplification solution is passed through are arranged so that subsequent zones are arranged next to each other, e.g. in a linear arrangement or in a meander-like arrangement or in a matrix arrangement. A meander-like arrangement is e.g. illustrated in appended figures 10 and 12, a linear arrangement is for example shown in appended figure 11, whereas a matrix-like arrangement is demonstrated in appended figure 13. Also combinations of different arrangements are possible. A meandering arrangement is a space-saving arrangement. Particularly in those embodiments in which the temperature of all zones or all zones of at least one kind is adjusted and maintained independently from each other, the arrangement of the zones can be customized for a particular application. Some embodiments also relate to arrangements of zones, where the temperature of all zones of only one kind of zone, e.g. the surface hybridization zone, is controlled, adjusted and maintained concertedly. A concerted control, adjustment or maintenance of the temperatures is e.g. realized by arranging all zones of the same kind next to each other without thermally decoupling them from each other. The arrangement of the temperature zones may in some embodiments be reconfigurable; in other embodiments it may be fixed. Particularly in a matrix layout, the arrangement of the temperature zones may be reconfigurable. Also the arrangement of the heater and/or coolers may be reconfigurable in some embodiments. Particularly the layout of heating electrodes may be fixed or reconfigurable.

Preferably, in the methods according to the present invention multiple nucleic acid sequences are detected by at least one capture probe complementary to each target nucleic acid sequence to be detected.

The capture probe may for example be an oligonucleotide probe.

Multiple target nucleic acids in the context of the present invention may be any number of different target nucleic acids detectable simultaneously or in parallel, particularly but not limited to 2, 3, 4, 5, 6, 7, 8, 9, 10, 11, 12, 13, 14, 15, 16, 17, 18, 19, 20, 21,22,23,24,25,26,27,28,29,30,31,32,33,34,35,36,40,4

5,48,50,52,56,55,60,64, 65, 70, 72, 75, 80, 85, 90, 95, 96, 100 or 384 target nucleic acids. Multiple target nucleic acids hybridized to capture probes may be detected simultaneously or in parallel.

The capture probes may be for example immobilized on an inner surface of said surface hybridization zone or on the surface of beads. The beads may e.g. be magnetic beads.

In some particular embodiments, for each distinct target nucleic acid sequence at least one pair of primers is used for amplification, wherein at least one primer of the pair of primers is labeled, e.g. with a fluorescent dye. In these cases fluorescence is detected on the surface of the surface hybridization zone. In other embodiments general primers (also known as consensus primers) are used to amplify different targets.

Preferably, the surface hybridization zone has a temperature allowing for hybridization of said capture probes to amplified target nucleic acid sequences and wherein said temperature is substantially constant.

In a particular embodiment, the temperature of the surface hybridization zone is increased after hybridization to measure melting and/or melting curves.

In some embodiments of the method, surface hybridization and/or detection is performed during and/or after each amplification cycle or during and/or after each N$^{th}$ amplification cycle, wherein N is 2, 3, 4, 5, 6, 7, 8, 9 or 10.

Particularly, the detection may be performed during and/or after each amplification cycle, however not during the first 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, preferably the first 20, more preferably the first 30 amplification cycles.

Preferably, the surface hybridization zone has a temperature allowing for hybridization of capture probes, particularly oligonucleotide probes, to amplified target nucleic acid sequences, wherein said temperature is substantially constant.

The capture probes are preferably surface-immobilized oligonucleotide probes complementary to said target nucleic acid sequences.

Even more preferably, the capture probes are surface-immobilized oligonucleotide probes complementary to said target nucleic acid sequences and the detection of the amplified target nucleic acid sequences captured to the captured sites is performed with a surface-specific detection method detecting a signal in proximity to the surface.

Alternatively, e.g. in the case of an embodiment with a meandering structure as outlined above and illustrated e.g. in Fig. 10 and 12, it is also possible to include an optional washing step to wash away the non-hybridized nucleic acids and primers and performing a detection afterwards. This way, a curve with the concentration of hybridized nucleic acids after a defined hybridization time (which can be unique for each cycle) vs. the number of amplification cycles (q=1..N) is obtained.

In some embodiments, the temperature in the surface hybridization zone is about the temperature allowing for annealing of primers to the nucleic acid sequences.

The nucleic acid sequences to be amplified and detected may preferably be selected from the group comprising ssDNA, dsDNA, RNA. However, all kinds of nucleic acids which in principle can be amplified may be amplified in the context of the present invention.

The nucleic acid polymerase according to the present invention may be a thermostable nucleic acid polymerase e.g. from a thermophilic organism. The nucleic acid polymerase may preferably be a DNA polymerase. The polymerase may be recombinant. Also polymerases adapted for hot-start-PCR may be used.

The thermostable nucleic acid polymerase may be for example selected from the group comprising *Thermus thermophilus* (Tth) DNA polymerase, *Thermus acquaticus* (Taq) DNA polymerase, *Thermotoga maritima* (Tma) DNA polymerase, *Thermococcus litoralis* (Tli) DNA polymerase, *Pyrococcus furiosus* (Pfu) DNA polymerase, *Pyrococcus woesei* (Pwo) DNA polymerase, *Pyrococcus kodakaraensis* KOD DNA polymerase, *Thermus filiormis* (Tfi) DNA polymerase, *Sulfolobus solfataricus* Dpo4 DNA polymerase, *Thermus pacificus* (Tpac) DNA polymerase, *Thermus eggertsonii* (Teg) DNA polymerase and *Thermus flavus* (Tfl) DNA polymerase.

A very particular embodiment, relates to a method for amplification and detection of target nucleic acid sequences, wherein the target nucleic acid is a doublestranded DNA sequence (dsDNA), wherein a pair of primers is used for amplification of the dsDNA, wherein one primer is substantially complementary to a region on one strand of the dsDNA and the other primer is substantially complementary to a region on the other strand of the dsDNA,
and wherein one primer is used in excess to the other primer, and wherein the melting point of the primer used in excess is at least 5°C lower than the melting point of the other primer.

In some embodiments the polymerase chain reaction (PCR) is an asymmetric PCR or is a Linear-After-The-Exponential-PCR (LATE-PCR).

Asymmetric PCR in this context is a PCR in which the concentration of the forward primer is different from the concentration of the reverse primer in the amplification solution, i.e. one primer is used in a great excess over the other primer. This results in a higher amount of single-stranded amplified DNA as compared to conventional, symmetric PCR, since the strand to which more primer is annealed to is preferentially amplified. Higher amounts of single-strand DNA may result in higher detection efficiencies.

Linear-After-The-Exponential-PCR (LATE-PCR), uses a limiting primer with a higher melting temperature than the excess primer to maintain reaction efficiency as the limiting primer concentration decreases mid-reaction.

The composition of amplification solutions allowing for amplification reactions such as PCR are known to a skilled person.

Herein, the temperature allowing for denaturation of the target nucleic acid is preferably in the range of from about 85 to 100 °C; the temperature allowing for primer annealing to said target nucleic acid is preferably in the range of from about 40 to 65 °C and the temperature allowing for primer extension is preferably in the range of from about 60 to 75 °C.

The amplification solution may be a homogeneous solution (e.g. PCR master mix) or may comprise two or more components: e.g. a PCR master mix and/oil or air.

The reaction container according to some embodiments of the invention may comprise a micro-array at least in said surface hybridization zone, wherein said capture probes, e.g. oligonucleotide probes, are immobilized on an inner surface of said micro-array.

The methods of the present invention may in some embodiments comprise the step of cooling of the amplification solution after the last amplification step (e.g. after the last PCR cycle) to a temperature below the hybridization temperature, preferably a cooling temperature in the range of from about 0°C to 10°C, more preferably in the range of from about 0 °C to 4 °C, most preferably around 3 °C to 4°C. In some embodiments cooling may be performed in the thermocycler zone in other embodiments cooling may be performed in a designated cooling zone that is thermally decoupling from the other zones.

The methods of the present invention are especially suitable for detection of nucleic acid comprising target nucleic acid sequences in said amplification solution. Thus, the amplification solution may be a solution suspected to comprise nucleic acids containing said target nucleic acid sequences.

The particular embodiments of the methods according to the present invention are also reflected in particular embodiments of the devices of the present invention, i.e. the present invention also relates to a device for conducting the methods according to the present invention. The definitions of specific terms described herein for the methods of the invention also apply for the devices of the present invention. This is particularly true for the concept of the zones and kinds of zones, and for the capture probes, reaction container, target nucleic acid (sequence) and the like.

The present invention also relates to a device for amplification and detection of target nucleic acid sequences in an amplification solution in a reaction container, comprising a reaction container for receiving an amplification solution comprising said target nucleic acid sequences, wherein the reaction container comprises at least one surface hybridization zone in which capture probes are immobilized on a surface, wherein said capture probes are substantially complementary to regions on said target nucleic acid sequences; one or more temperature controllers and/or temperature adjusters for generating a temperature profile of at least two kind of temperature zones in said reaction container, wherein one kind of zone is essentially identical to said surface hybridization zones, and wherein the surface hybridization zone has a substantially constant generated temperature allowing for hybridization of capture probes to complementary target nucleic acid sequences and wherein the temperature controllers and/or temperature adjusters control, adjust and maintain the temperature in the zones; a detection system that detects target nucleic acid sequences which are bound to said capture probes but does essentially not detect target nucleic acid sequences which are not bound to said capture probes; and a transportation system for transporting the amplification solution between the zones.

The reaction container comprises at least one other zone which may have a different generated temperature as the surface hybridization zone.

In a further aspect, the present invention also relates to a device for amplification and detection of target nucleic acid sequences in an amplification solution in a reaction container, comprising a reaction container comprising at least two kind of zones that are thermally decoupled from each other, wherein one kind of zone is a surface hybridization zone having a substantially constant generated temperature allowing for hybridization of capture probes to complementary target nucleic acid sequences, wherein an inner surface of the surface hybridization zone is coated with capture probes for target nucleic acid sequences; a detection system that detects targets which are bound to said capture probes; one or more temperature controllers and one or more temperature adjusters for controlling, adjusting and maintaining the temperature in the zones; and a transportation system for transporting the amplification solution between the zones.

The reaction container comprises at least one compartment for containing the amplification solution. Optionally, the reaction container comprises additionally fluidic elements such as fluid channels for transporting the amplification fluid between the said compartments or pumps for defining a flow through the said fluid channels.

In preferred embodiments of the device of the invention, the reaction container is comprised in a cartridge which may exchangeably be attached to the rest of the device. Such a cartridge may be a cartridge for the use in a thermocycler or in a device according to the present invention. The cartridge may in preferred embodiments be a disposable (one-way) cartridge. The temperature controllers and adjusters, e.g. the heaters and/or coolers are preferably not part of such a cartridge, particularly of a disposable cartridge. In alternative embodiments heaters and/or coolers may be part of the cartridge. The cartridge may comprise additional compartments, e.g. for preparation of the amplification solution (i.e. preparation of the sample). Also comprised in such a cartridge may in some embodiments be the transportation systems or parts of the transportation systems, e.g. microfluidic systems. The structure or pattern of the zones of the reaction chamber may be reflected in the structure or pattern of the heaters and/or coolers of the temperature control and adjustment system. Also the cartridge may have patterns of heat conducting and/or insulating surfaces.

It is preferred that the reaction container is comprised in an exchangeable cartridge. As stated above, the temperature controllers and/or adjusters or parts thereof are in some embodiments comprised within the cartridge or is in other embodiments not comprised within the cartridge. The same applies, independently, to the transportation system. The transportation system or parts thereof is comprised within the cartridge or is not comprised within the cartridge.

The temperature zones of the reaction container are a product of the layout of the temperature control and adjustment system, i.e. the arrangement of the heaters, coolers, heat transfer means and the like. If a cartridge is present, the layout of the zones is dependent on the design of the cartridge and the arrangement of the heaters, coolers and heat transfer means. Thus, in some embodiments the zone layout within the cartridge is defined by the layout of the temperature controllers and adjusters outside the cartridge.

Preferably, the detection system detects targets which are bound to said capture probe but does essentially not detect targets which are not bound to said capture probe.

Depending on the type of transport, the transportation system may be an active or passive system. The transportation system in this context may e.g. be a passive system inherently comprised in said reaction container, e.g. in cases where the transport is solely based on physical effects such as convection or diffusion. The transportation system may in some embodiments comprise a Microelectromechanical system (MEMS), one or more capillaries, one or more pumps and the like or combinations thereof.

The device of the present invention may preferably be a device for amplification and detection of target nucleic acid sequences in a polymerase chain reaction (PCR).

Preferably, said detection system is a surface-specific detection system that detects targets which are bound to said surface but does essentially not detect targets which are in solution.

Optionally, said device may comprise a washing means to wash away unbound or unspecifically bound nucleic acid sequences, particularly nucleic acid sequences that are not bound to capture probes or that are unspecifically bound. A washing means may for example be the possibility to pump or transfer a washing buffer through the reaction container or surface hybridization zone or parts thereof. Therefore an additional container containing said washing buffer may be present.

Preferably, the detection is a real-time detection and the PCR is a real-time PCR. More preferably, the PCR is a quantitative PCR and the amplified target nucleic acid sequences are quantified. Real-time detection in this context means that amplified nucleic acid sequences may be detected during amplification, e.g. during and/or after each amplification cycle or during and/or after defined or predetermined amplification cycles.

The temperature controllers and adjuster may for example be heaters and/or coolers, preferably with a temperature detector and/or a feedback-regulation system. The heaters may in some embodiments be e.g. Peltier elements, heating electrodes, hot/cool air devices, water baths and the like. However, the invention is not limited to these heaters/coolers and all kind of heating and cooling means may be used for controlling, adjusting and maintaining the temperature of the zones. Also combinations of different heaters and/or coolers may be used. The temperature controller and adjusters may also comprise means for transferring or conducting heat, e.g. from the heater to the reaction chamber.

In one embodiment of the device at least one surface hybridization zone is used for hybridization and detection of amplified target nucleic acid sequences at more than one stages of the entire amplification process.

In the context of this particular embodiment of the device, preferably, only one surface hybridization zone for the detection of the amplified target nucleic acid is present in the reaction container.

Two or more of the at least two thermally decoupled zones may be comprised in the same compartment or volume of the reaction container or the at least two thermally decoupled zones may be comprised in separate compartments or volumes of the reaction container.

In a particular embodiment, at least one surface hybridization zone and at least one thermocycler zone of which the temperature can be cycled in the range of from the melting point (melting or freezing temperature) to the boiling point (boiling temperature) of the amplification solution (preferably within the range of from about 0°C to 100°C) is present. Particular layouts of embodiments of the device according to the present invention comprising a thermocycler zone are illustrated in appended figures 2 to 4. The thermocycler zone is reflected in the design of the temperature control and adjustment system which in this embodiment is a system allowing to cycle the temperature of the thermocycler zone in the reaction container.

In another embodiment the reaction container of the device comprises at least one surface hybridization zone and at least one denaturation zone. Such an embodiment is for example illustrated in appended figures 6 and 7.

In yet another embodiment the reaction container of the device comprises at least one surface hybridization zone, at least one generated denaturation zone and at least one generated extension zone. Such an embodiment is for example illustrated in appended figure 5.

The amplification solution may be transported unidirectional or bidirectional between the zones.

Thus, in some embodiments of the device, the amplification solution may be transferred between the thermocycler zone and the surface hybridization zone in a circular manner (unidirectional) (Fig. 4 and Fig. 7) or in a back-and-forth manner (bidirectional) (Fig. 3).

The zones or the zones of one kind (e.g. the surface hybridization zones) may in some embodiments be in a channel-like form.

Preferably, the zones may, depending on the reaction volume and the particular application, have dimensions in the range of from about (but not limited to) a height of the volume above the zones between about 1-500 μm, and width and length of the zones between about 100 μm - 1 cm.

In a further embodiment the present invention relates to a device in which a plurality of surface hybridization zones is present in the reaction container such that when in use, at two or more stages of the entire amplification process surface hybridization may occur.

In this context, hybridization and detection may preferably occur during and/or after a plurality of distinct or defined amplification cycles, most preferably during and/or after each amplification cycle.

Furthermore, the present invention also relates to a device for amplification and detection of target nucleic acid sequences in an amplification solution in a reaction container,
wherein the reaction container comprises three kinds of thermally decoupled zones,
wherein the generated temperature in each zone may be kept substantially constant,
and wherein the first kind of zone is an annealing zone, the second kind is a denaturation zone and the third kind is an extension zone,
and wherein for each amplification cycle for which detection is desired one surface hybridization zone is present or the annealing zone is substantially overlapping with the surface hybridization zone,
wherein the amplification solution is passed through all zones such that for each amplification cycle first a denaturation zone, secondly an annealing zone and thirdly an extension zone is passed,
wherein the transport is unidirectional and non-circular, and wherein the denaturation zones have a generated temperature allowing for denaturation of the target nucleic acid sequences, the annealing zones have a temperature allowing for annealing of primers, the surface hybridization zones have a generated temperature allowing for annealing of primers and hybridization of a capture probe and the extension zones have a generated temperature allowing for primer extension.

In a particular embodiment of this device, for each amplification cycle one hybridization zones is present and the number of surface hybridization zones is equal to the number of denaturation zones and extension zones. Particular embodiments of such a device are illustrated in the appended figures 10 to 13.

In one embodiment of the device, the temperature of the reaction container is controlled concertedly for each member of one kind of zone (see for example appended Fig. 10).

In another embodiment of the device, the temperature of the reaction container is controlled separately for each zone (see for example appended figures 11 to 13).

Fig. 11 shows a schematic layout of a particular embodiment of the device according to the present invention, it comprises individually addressable temperature zones for denaturation (1), hybridization and primer annealing (2), and elongation (3), an unidirectional, noncirculating flow channel (11) such that for each cycle (N, N+1, N+2, ...) the PCR fluid passes the individually addressable temperature zones for that particular cycle; where the flow runs from the left to the right, a dedicated hybridization zone (20) for each cycle (in principle the hybridization can also be skipped for one or more particular cycles) with capture probe (21).

As an alternative, see e.g. Fig. 10, Fig. 12 and Fig. 14, also a meandering flow can be used (10) in order to have the distance between hybridization zones of the PCR cycles as small as possible, which is advantageous from a detection point of view; the larger the distance between the hybridization zones for adjacent PCR cycles the faster scanning (in case of a scanning optical reader) or the larger field of view (in case of an imaging optical reader) is needed. In Fig. 12, the order of the temperature zones is (1), (2), (3), but any combination (from bottom to top) of the 3 temperature zones where denaturation is performed just before the annealing and hybridization zone can be used: (1), (2), (3); (3), (1), (2); (3), (2), (1); (2), (1), (3). To adapt the denaturation, hybridization, and annealing times it is of course possible to change the shape of the meander (e.g. multiple passes over the hybridization zone before flow is directed towards elongation zone). The shape of the flow channel itself is not relevant for the operation except that it determines the times that the fluid experiences the temperature steps. In principle there is no need for having the flow channel running in a single plane. It is not necessary to have hybridization zones for each cycle, as an illustrative example, in the case of a start copy number of 100000 in a volume of 25 micro liter (molar concentration of 6.6 fM), for a threshold concentration (given the sensitivity and measurement time available) of 0.1 nM this requires 14 PCR cycles (assuming 100% PCR efficiency) to have a signal above threshold. In case the start copy numbers are below 100000, no hybridization zones are needed for the first 14 cycles. However, the above exemplary considerations are not limiting the scope of the invention. To make the device suitable for any layout of the cartridge (fluid channel) one can extend the heater layout to a 2D matrix, as illustrated in e.g. Fig. 13.

The temperature zones in Figs. 11 to 13 can also be composed of multiple heaters in order to create a temperature profile along the fluid channel.

In a preferred embodiment which is illustrated in appended Figures 14 to 16, the reaction chamber is comprised in a cartridge (50) and the temperature zones are arranged in a meander-like pattern. The body housing (51) of the cartridge preferably comprises an at least partly transparent material to enable optical detection. The cartridge is preferably made of (but is not limited to) plastic and like materials or glass. The cartridge can also be an assembly of a transparent substrate with capture probes and a box containing fluid channels. Capture probes (20) that are substantially complementary to target nucleic acid sequences are immobilized in hybridization zones. Typically, but by no means limiting, the capture probe sites are spots 0.1 mm in diameter. The cartridge of this embodiment preferably also comprises an inlet for fluid (40), a fluid channel with a meandering layout (41) and an outlet for the fluid (42). Typical preferred dimensions of the fluid channel are a height of in the range of about 0.05-1 mm and a width of in the range of about 0.1-1 mm. As mentioned above, the cartridge in this preferred embodiment may be exchangeable. The device is illustrated in Fig. 15 without the cartridge and in Fig. 16 with the cartridge. The device of Fig. 15 is ready to receive a (disposable) cartridge with the reaction chamber. The device comprises a sample holder (70) for receiving the cartridge (50), heaters (80) (or coolers or other temperature adjusters) for defining the temperature in the temperature zones (1)-(3) of the cartridge. In particular cases there are three heaters (80) present to define the temperatures in zones (1, denaturation zone), (2, annealing/hybridization zone), (3, extension zone). A detection system ("reader") for detecting the target nucleic acids that are hybridized to the capture probes (20) is also present. A simplified schematic illustration of a scanning unit for confocal detection is shown in Fig. 15 and 16. In a particularly preferred case the scanning unit comprises an illumination/collection (of the fluorescence) lens/objective (92) and a dichroic mirror (91) that reflects the excitation light (101 in Fig. 16) resulting in excitation light directed towards the cartridge. The same lens (92) collects in this case the fluorescence (201 in Fig. 16) generated by the labeled target molecule and directs this fluorescence via the dichroic mirror (91), which transmits the fluorescent light, towards a detector (not shown in the figures). It is of course straightforward for someone skilled in the art to replace the meandering configuration of this exemplary embodiment with another configuration according to the invention.

The zones of the devices according to the present invention may in some embodiments be connected to each other through a valve or a valve-like structure or through a tap.

The device may also comprise a controller for opening or closing the tap or valve and an opening or closing mechanism for the tap or valve. By opening and closing the tap or valve and by the time of opening the valve or tap, the transfer of the amplification solution between the zones, particularly between thermocycler zone and surface hybridization zone and between denaturation zone and surface hybridization zone and between surface hybridization zone and extension zone and between extension zone and denaturation zone may be controlled.

The transportation system of the device is preferably selected from the group comprising a pump, a heating element and a Microelectromechanical system (MEMS).

The transport may be an active transport, e.g. by applying a pressure with a pump or may be a passive transport, e.g. diffusion or transportation driven by capillary forces. Thus, when the amplification solution is passed through the zones, this may imply an active as well as a passive transport through the zones.

By controlling the transportation system, e.g. the rate of transportation and/or the periods where the transportation system is active, the amplification and detection can be further controlled. The rate of transportation, e.g. the flow through the zones (e.g. in terms of volume per time) or the time the amplification solution stays in a particular zone may be controlled and adjusted according to a particular application. A skilled person is for instance aware of the timing of PCR cycles and the phases of each cycle.

The detection system of the device may preferably be a confocal or evanescent detection system and/or is selected from the group of fluorescence detection system, CCD chip, plasmonics (e.g. a surface plasmon resonance system), total internal reflection system and wire grid biosensor system.

In some embodiments, the device comprises a micro-array at least in said surface hybridization zone and said capture probes are immobilized on an inner surface of said micro-array.

In preferred embodiments of the device, the device comprises multiple different capture probes substantially complementary to multiple target nucleic acid sequences and a detection system for simultaneously detecting multiple different targets.

The device may additionally comprise a cooling zone for cooling the amplification solution to a temperature below the hybridization temperature, preferably a temperature in the range of from about 0 °C to 10 °C, more preferably in the range of from about 0 °C to 4 °C, most preferably around 3 °C to 4°C. The amplification solution may for example be transferred to the cooling zone after the last PCR cycle.

The present invention also relates to a cartridge for amplification and detection of target nucleic acid sequences in an amplification solution in a reaction container, comprising a reaction container for receiving an amplification solution comprising said target nucleic acid sequences, wherein the reaction container comprises at least one surface hybridization zone in which capture probes are immobilized on a surface, wherein said capture probes are substantially complementary to regions on said target nucleic acid sequences. The cartridge may be a disposable cartridge. Preferably, the reaction container comprises at least two surface hybridization zone in which capture probes are immobilized on a surface, wherein said capture probes are substantially complementary to regions on said target nucleic acid sequences. The features of some illustrative embodiments of the cartridge have already been discussed herein above in context of the cartridge as a component of the device of the present invention. A particular embodiment of such a cartridge is illustrated in appended figure 14. When inserted into the device the surface hybridization zone in the cartridge preferably overlaps with the zone of the device in which a temperature allowing for hybridization is generated.

Thus, the present invention also relates to a device for receiving the cartridge of the present invention, wherein the device comprises:

one or more temperature controllers and/or temperature adjusters for generating a temperature profile of at least two kind of temperature zones in a reaction container comprised in said cartridge, wherein one kind of zone has a substantially constant generated temperature allowing for hybridization of capture probes to complementary target nucleic acid sequences and wherein the temperature controllers and/or temperature adjusters control, adjust and maintain the temperature in the zones;
a detection system that detects targets which are bound to said capture probes but does essentially not detect targets which are not bound to said capture probes;
a transportation system for transporting the amplification solution between the zones; and
a receiving element for said cartridge.

It is preferred that when a cartridge inserted into the device the surface hybridization zone of the cartridge overlaps with the zone of the device in which a temperature allowing for hybridization is generated.

The present invention also relates to the use of the methods, cartridges and devices of the present invention for quantitative analysis of target nucleic acid sequences, for simultaneous quantitative analysis of multiple target nucleic acid sequences or for analyzing a sample for the presence of a target nucleic acid.

Preferably, the methods, cartridges and devices described herein are used for clinical diagnosis, point-of care diagnosis, bio-molecular diagnostics, gene or protein expression arrays, environmental sensors, food quality sensors or forensic applications.

The present invention also relates to the use of the methods, cartridges and devices of the present invention in PCR, quantitative PCR, multiplex PCR, real-time PCR or real-time multiplex PCR, particularly quantitative real-time PCR or quantitative real-time multiplex PCR.

The following illustrations further specify some aspects of the invention in more detail:

Amplification may be performed by various enzymatic methods including PCR (polymerase chain reaction), NASBA (nucleic acid sequence based amplification), TMA (transcription mediated amplification), and rolling circle amplification. Enzymatic amplification methods suitable for the present invention are known to a person skilled in the art.

Amplification of target nucleic acid sequences according to the present invention is performed in an amplification solution. The amplification solution may in the context of the present invention for example be a sample containing or suspected to contain nucleic acids comprising target nucleic acid sequences. A skilled person knows how to prepare a sample for performing an amplification reaction therein or knows additional components (e.g. buffers, enzymes, nucleotides, salts etc.) that have to be added to a sample in order to perform an amplification reaction in a sample. An amplification solution herein may also relate to a reaction buffer comprising nucleotides (e.g. dNTPs) and other substances (e.g. buffering agents, salts like magnesium salts, inert proteins like BSA) allowing for amplification of target nucleic acids. A skilled person knows suitable solutions (and particularly suitable concentrations of the ingredients of such solutions) for amplification reactions, particularly polymerase chain reactions (PCR). PCR comprises the repetition of cycles comprising a denaturation phase of the nucleic acid to be amplified (termed "target nucleic acid" or "target nucleic acid sequences" herein) at a high temperature well above the melting temperature of the target nucleic acid sequence, an annealing phase at a temperature allowing for annealing of nucleic acid primer to said target nucleic acid and a primer extension phase (elongation phase) at a temperature allowing for primer extension by a nucleic acid polymerase in which the annealed primer(s) is/are extended and the target nucleic acid serves as a template. The skilled person is aware that for polymerase chain reaction suitable primers or pairs of primers are present during amplification in the amplification solution and knows how to design such primers in order to amplify target nucleic acid sequences.

A primer is a nucleic acid strand, or a related molecule that serves as a starting point for nucleic replication. The term "nucleic acid polymerase" refers to an enzyme that synthesizes nucleic acid stands (e.g. RNA or DNA) from ribonucleoside triphosphates or deoxynucleoside triphosphates.

A thermally decoupled zone in the context of the present invention relates to a zone or a compartment of a reaction container in which the temperature can be controlled, adjusted and maintained essentially independently from other zones or compartments or other kinds of zones or compartments of the reaction container. Zones of the same kind may preferably be thermally decoupled from each other. However, as described herein below in more detail, in some embodiments, zones of the same kind of zone may be thermally coupled or alternatively in other embodiments be thermally decoupled from each other, i.e. the temperature of zones of the same kind may be controlled, adjusted and maintained concertedly or independent from each other. That may in some embodiments mean that zones of the same kind have about the same temperature, in other embodiments it may mean that the temperature may vary among the zones of the same kind. According to the present invention, the temperatures of the different kinds of zones are controlled, adjusted and maintained independently from other kinds of zones such that the different kinds of zones are thermally-decoupled. In some embodiments the thermally decoupled zones are physically separated and/or isolated compartments, i.e. their volumes are split. In other embodiments two or more thermally decoupled zones may be comprised in the same compartment and/or may share the same volume provided that the temperatures of the zones in the same volume or compartment can be adjusted and maintained separately, e.g. by separate heaters or coolers.

A kind of zone is a class of zones which serve for the same purpose. Kinds of zones are, according to the present invention, e.g. surface hybridization zones, thermocycler zones, denaturation zones, extension zones and/or annealing zones. The temperature of the hybridization zones, the extension zones, the annealing zones and the denaturation zones are substantially kept constant in the preferred embodiments of the invention during amplification and/or detection. The temperature of the thermocycler zones is changed, e.g. cycled between two, three, four or more distinct temperatures in a programmable manner during the amplification reaction. A skilled person knows how to select and/or program thermocycles for e.g. polymerase chain reaction, e.g. how to select the times and temperatures of the phases and the number of cycles. Preferably, the temperature in a thermocycler zone is cycled between denaturation temperature, annealing temperature and extension temperature. In some embodiments, e.g. for hot-start PCR, additional temperature phases and/or cycles may be included in thermo-cycling, e.g. before the first cycle or after the last cycle. The temperature in the denaturation zone (s) is the denaturation temperature, i.e. a temperature allowing for denaturation of the target nucleic acid sequence. The temperature in the annealing zone(s) is the annealing temperature, i.e. a temperature allowing for annealing of primers to the target nucleic acid sequence. The temperature in the extension zone(s) is the extension temperature, i.e. a temperature allowing for primer extension by a nucleic acid polymerase, wherein the target nucleic acid sequence serves as a template. The temperature in the surface hybridization zone(s) is the hybridization temperature, i.e. a temperature allowing for hybridization of capture probes, (oligonucleotide probes) to the target nucleic acid sequence. These temperatures depend on the properties (e.g. length or GC content) of the target nucleic acids, primers, polymerase and oligonucleotide probes (capture probes) and a skilled person knows how to select, determine or calculate these temperatures. Typical denaturation temperatures are in the range of from about 90 to 99 °C, preferably of from about 94 to 98°C. Typical annealing temperatures are in the range of from about 50 to 70 °C, preferably of from about 55 to 68 °C. Typical extension temperatures are in the range of from about 70 to 80 °C, preferably of from about 72 to 75 °C. Typical hybridization temperatures are in the range of from about 40 to 70 °C, preferably of from about 45 to 68 °C.

According to the present invention, surface specific detection is obtained either by a surface specific detection system and/or by surface-specific generation of the signal to be detected.

The following definitions apply to this and the other embodiments of the invention:

Surface specific detection means that the contribution to the detected signal by amplicons that are not captured (e.g. floating in the fluid on top of the surface with capture probes) is substantially suppressed, which means preferably suppressed by at least a factor 50, more preferably by at least a factor 100, and even more preferably by at least a factor 1000, while the contribution to the detected signal by captured amplicon is (substantially) not suppressed.

For fast hybridization the following boundary conditions may apply on the part of the PCR chamber containing the capture probes: The width of the channel is proportional to the spot lateral size and the height of the channel is small (preferably about 100-300 μm).

These boundary conditions insure that the whole sample is passed along the capture probes and in combination with the small height of the channel provide fast hybridization of the amplicons to the capture probes.

After each PCR cycle the amount of amplicons substantially doubles and therefore the amount of hybridized amplicons increases proportionally. The amount of bound amplicons is proportional to its concentration in the solution according to well-known formula

$$h = \frac{k_f c_A C_P}{k_f c_A + k_r} \left(1 - e^{-(k_f c_A + k_r)t}\right)$$

Where:

h - is the surface concentration of hybridized complex to the capture sites in (mol/m$^2$),
$c_A$ - analyte concentration (mol/m$^3$)
$C_P$ - capture probe surface concentration (mol/m$^2$)
and $k_f$ and $k_r$ are the on and off rates of binding.

Fig. 9 illustrates that in the beginning of the hybridization curve the amplicon concentration is proportional to the slope of the hybridization curve as explained and therefore by measuring hybridized signal during each PCR cycle quantitative and real-time detection of the hybridization of amplicons can be realized.

As an example the case of a fluid cell having a height of 500 microns and containing labeled primers at a concentration of 1 micromolar is considered:

1) Without surface specific detection, the labeled primers give rise to a detected signal equivalent to ~300000 labels per square micrometer. For a capture probe density of 10000 capture probes per square micrometer, in the best case this would result in a signal over background ratio of 1/30. For a typical hybridization experiment not all capture probes have bound to a labeled amplicon and the signal over background ratio will be even smaller; e.g., 1/300 for 1000 captured amplicons per square micrometer.

2) With surface specific detection, the labeled primers in solution give rise to a substantially lower detected signal; e.g., detected signal equivalent to -300 labeled primers per square micrometer for a background suppression factor of 1000. With surface-specific detection, the signal over background ratio is substantially improved to about 3 for 1000 captured labeled amplicons per square micrometer.

Another way to describe surface specific detection is to describe a surface-specific detection method or device that detects those labels which are bound to the surface but does essentially not detect labels which are in solution. This means that the above definition given for surface specific detection applies equally to the term "a surface-specific detector that detects those labels which are bound to the surface but does essentially not detect labels which are in solution"

The capture probe molecule can be a DNA, RNA, PNA (peptide nucleic acid), LNA (locked nucleic acid), ANA (arabinonucleic acid), or HNA (hexitol nucleic acid) oligonucleotide. RNA, PNA, LNA, and HNA are able to form heteroduplexes with DNA that are more stable that DNA:DNA homoduplexes. This ensures enhanced discrimination ability for sequence mismatches (more specific hybridization). The higher stability of heteroduplexes also allows the use of shorter oligonucleotide probes at a given temperature reducing the chance of non-specific binding. PNA:DNA duplexes are formed independent of ionic strength of the hybridization buffer. This may enhance the hybridization efficiency in low salt PCR buffers.

Hybridization of a portion of the amplicons means that the concentration of amplicon can be directly calculated from the intensity of the signal measured due to hybridization of amplicons to the capture probes. If the relation between the measured signal and amplicon concentration is not linear, a correction algorithm or calibration curve may be applied in order to deduce the amplicon concentration.

The capture portion of the capture probe may contain from 10 to 200 nucleotides, preferably from 15 to 50 nucleotides, more preferably from 20 to 35 nucleotides specific for the amplicons produced during the amplification reaction. The capture probe can also contain additional nucleotide sequences that can act as a spacer between the capture portion and the surface or that can have a stabilizing function that can vary from 0 to 200 nucleotides, preferably from 0 to 50. These non-capturing nucleotide sequences can either contain normal nucleotides or a-basic nucleotides.

The capture molecule may be immobilized by its 5' end, or by its 3' end.

For multiplexing, capture molecules are immobilized in specifically localized areas of a solid support in the form of a micro-array of at least 4 capture molecules per $\mu m^2$, preferably at least 1000 capture molecules per $\mu m^2$, more preferably at least 10000 capture molecules per $\mu m^2$, and even more preferably 100000 capture molecules per $\mu m^2$.

In a specific embodiment, the capture molecules comprise a capture portion of 10 to 100 nucleotides that is complementary to a specific sequence of the amplicons such that said capture potion defines two non-complementary ends of the amplicons and a spacer portion having at least 20 nucleotides and wherein the two non-complementary ends of the amplicons comprise a spacer end and a non-spacer end, respectively, such that the spacer end is non-complementary to the spacer portion of the capture molecule, and said spacer end exceeds said non-spacer end by at least 50 bases.

The terms "nucleic acid, oligonucleotide, array, nucleotide sequences, target nucleic acid, bind substantially, hybridizing specifically to, background, quantifying" are the ones described in the international patent application WO 97/27317 incorporated herein by reference. The term polynucleotide refers to nucleotide or nucleotide like sequences being usually composed of DNA or RNA sequences.

The terms "nucleotides triphosphate, nucleotide, primer sequence" are further those described in the documents WO 00/72018 and WO 01/31055 incorporated herein by references.

References to nucleotide(s), polynucleotide(s) and the like include analogous species wherein the sugar-phosphate backbone is modified and/or replaced, provided that its hybridization properties are not destroyed. By way of example, the backbone may be replaced by an equivalent synthetic peptide, called Peptide Nucleic Acid (PNA).

According to a preferred embodiment the capture probes are immobilized on the hybridization surface in a patterned array. According to a preferred embodiment the capture probes are immobilized on the surface of micro-arrays.

"Micro-array" means a support on which multiple capture molecules are immobilized in order to be able to bind to the given specific target molecule. The micro-array is preferentially composed of capture molecules present at specifically localized areas on the surface or within the support or on the substrate covering the support. A specifically localized area is the area of the surface which contains bound capture molecules specific for a determined target molecule. The specific localized area is either known by the method of building the micro-array or is defined during or after the detection. A spot is the area where specific target molecules are fixed on their capture molecules and can be visualized by the detector. In one particular application of this invention, micro-arrays of capture molecules are also provided on different or separate supports as long as the different supports contain specific capture molecules and may be distinguished form each other in order to be able to quantify the specific target molecules. This can be achieved by using a mixture of beads which have particular features and are able to be distinguishable from each other in order to quantify the bound molecules. One bead or a population of beads is then considered as a spot having a capture molecule specific to one target molecules.

Micro-arrays are preferably obtained by deposition of the capture molecules on the substrate which is done by physical means such as pin or "pin and ring" touching the surface, or by release of a micro-droplet of solution by methods such as piezo- or nanodispenser.

Alternatively, in situ synthesis of capture molecules on the substrate of the embodiments of the inventions with light spatial resolution of the synthesis of oligonucleotides or polynucleotides in predefined locations such as provided by US 5,744,305 and US 6,346,413.

It may be preferred that the PCR mixture can be enriched for single stranded amplicons by means of asymmetrical PCR (or LATE PCR: linear after the exponential). In asymmetrical PCR, unequal concentrations of forward and reverse PCR primer are used. When the concentration of the labeled primer is higher than that of the unlabelled primer, the labeled strand will be amplified at a lower rate that the unlabelled strand. This not only leads to an accumulation of the labeled strand but also directly favors the hybridization of the labeled strand to the capture probe.

The term "real-time PCR" means a method which allows detection and/or quantification of the presence of the amplicons during the PCR cycles. In real-time PCR, the presence of the amplicons is detected and/or quantified in at least one of the amplification cycles. The increase of amplicons or signal related to the amount of amplicons formed during the PCR cycles are used for the detection and/or quantification of a given nucleotide sequence in the PCR solution. In a preferred embodiment, the presence of the amplicons is detected and/or quantified in every cycle.

The term "amplicon" in the invention relates to the copy of the target nucleotide sequences being the product of enzymatic nucleic acid amplification.

Instead of labeled PCR primers, internal labeling with labeled dNTPs can be used.

The label-associated detection methods are numerous. A review of the different labeling molecules is given in WO 97/27317. They are obtained using either already labeled primer, or by enzymatic incorporation of labeled nucleotides during the copy or amplification step (WO 97/27329).

Possible labels are fluorochromes which are detected with high sensitivity with fluorescent detector. Fluorochromes include but are not limited to cyanine dyes (Cy3, Cy5 and Cy7) suitable for analyzing an array by using commercially available array scanners (as available from, for example, General Scanning, Genetic Microsystem). FAM (carboxy fluorescein) is also a possible alternative as a label. The person skilled in the art knows suitable labels which may be used in the context of this invention.

In a preferred embodiment of the invention, a signal increase of the fluorescence signal of the array related to the presence of the amplicons on the capture molecule is detected as compared to the fluorescence in solution.

In a particular embodiment the differences of the detection of the fluorophore present on the array is based on the difference in the anisotropy of the fluorescence being associated with a bound molecule hybridized on the capture molecule as a DNA double helix compared to the fluorescence being associated with a freely moving molecule in solution. The anisotropy depends on the mobility of the fluorophores and the lifetime of the fluorescence associated with the fluorophores to be detected. The method assay for the anisotropy on array is now available from Blueshift Biotechnologies Inc., 38 East Caribbean Drive, Sunnyvale, CA 94089 (http://www.blueshiftbiotech.com/dynamicft.html).

In a particular embodiment, the detection of fluorophore molecules is obtained preferably in a timer-resolved manner. Fluorescent molecules have a fluorescent lifetime associated with the emission process. Typically lifetimes for small fluorophores such as fluorescein and rhodamine are in the 2-10 nanosecond range. Time-resolved fluorescence (TRF) assays use a long-lived (>1000ns) fluorophore in order to discriminate assay signal from short-lived interference such as autofluorescence of the matrix or fluorescent samples which almost always have lifetimes of much less than 10 ns. Lifetime is preferably modulated by the nearby presence of another fluorophore or a quencher with which a resonant energy transfer occurs. Instruments for TRF simply delay the measurement of the emission until after the short-lived fluorescence has died out and the long-lived reporter fluorescence still persists. Fluorescence lifetime can be determined in two fundamental ways. The time domain technique uses very short pulses (picosecond) of excitation and then monitors the emission in real-time over the nanosecond lifetime. Fitting the decay curve to an exponential yields the lifetime. The frequency domain technique modulates the excitation at megahertz frequencies and then watches the emission intensity fluctuate in response. The phase delay and amplitude modulation can then be used to determine the lifetime. The frequency technique for fast and economical lifetime imaging is now available from Blueshift Biotechnologies Inc. As stated above, theses definitions apply to all of the described embodiments.

In one embodiment of the invention, primers are labeled and the hybridized labeled amplicons are detected with a surface-specific detection system that detects those labels which are bound to the surface. Surface-specific means as defined above that labels which are in solution are essentially not detected.

In a preferred embodiment the signal of the label to be detected does not change in dependence of the binding state of said multiple nucleic acid sequences.

In an even more preferred embodiment the signal to be detected is a fluorescent signal. It is preferred that the label is a small organic fluorophore, but can also be a particulate label (either fluorescent or non-fluorescent), such as nano-phosphores, quantum dots.

For detection of multiple nucleic acids multiple labels may be used, in a preferred embodiment the same label is used for detection of each of said multiple target nucleic acid sequences.

The surface-specific detection system is preferentially selected from a group comprising a confocal measurement device, a plasmonic measurement device and a device for the measurement according to evanescent detection.

As mentioned above, to be able to discriminate between hybridized amplicons and primers or amplicons in solution, it is essential to make a surface specific measurement. A surface specific measurement only detects labels that are very close to the capture surface. Because hybridization can only take place where capture probes have been deposited and the PCR mixture is homogeneous, it is possible subtract the background (the fluorescence intensity between the spots) and to determine the amount of hybridized amplicons.

Possible labels are fluorescent labels or non-fluorescent (e.g. particulate) where a difference in refractive index or absorption can be detected by optical means. It should be straightforward for someone skilled in the art to know suitable fluorescent or non-fluorescent labels.

For highly surface specific measurements, that is a suppression of the background by at least a factor 50, one can distinguish between three different approaches:

1. Confocal: typical measurement height along the optical axis of 1-2 μm.
2. Plasmonic: measurement height of about the wavelength of excitation light or smaller.
3. Evanescent: measurement height of 100 nm or smaller.

**1. Confocal**

Confocal measurements can be made with various types of imaging equipment, e.g. a standard pinhole-based system. Such systems can be made very compact (PCT/IB2007/052499, PCT/IB2007/052634, and PCT/IB2007/052800). Different locations along the optical axis of the system give rise to different locations where the labels are imaged by the objective closest to the array surface. By using a pinhole and proper positioning -at the location where there is a sharp image of a label at the array surface- one can select a small measurement volume (depth along the optical axis of only 1-2 μm) in the neighbourhood of the sensor surface.

**2. Plasmonic**

Here the substrate is covered with a metal such as Au, Ag. The capture probes are on the metal layer or a spacing layer is deposited on top of the metal and subsequently covered with capture probes. The fluorescence of the labels of the hybridized DNA can couple to the surface plasmon at the metal medium/fluid interface. Labels in the fluid cannot couple or with a substantially smaller efficiency to the surface plasmon. By out coupling of the surface plasmon (that is converting the surface plasmon mode in to a propagating wave) and measuring the out coupled power, one essentially only measures the fluorescence of the labels of the hybridized DNA. As a result the fluorescence measurement is highly surface specific.

**3. Evanescent**

As another alternative method for enhancing the surface specificity, one can use evanescent excitation methods, where an evanescent wave is excited at the surface of the substrate and excites the fluorophores.

As a first method one can use total internal reflection (TIR) at the substrate-fluid interface, which results in measurement (excitation) volumes typically within 100-200 nm of the array surface. TIR however requires the use of a glass prism connected to the substrate or the use of a substrate with a wedge shape to enable the coupling of excitation light with angles above the critical angle of the substrate-fluid interface into the substrate.

Alternatively (as described in PCT/IB2006/051942, PCT/IB2006/054940) one can cover the substrate with a non-transparent medium such as a metal and pattern the metal with [an array of] apertures with at least one dimension in the plane parallel to the substrate-fluid interface below the diffraction limit of light in the fluid. As an example, one can pattern the substrate with wire grids that have one in-plane dimension above and the other dimension below the diffraction limit of the light in the fluid. This results in excitation volumes within 50 nm (Measurement volumes of 20-30 nm have already been demonstrated experimentally) of the array surface. Advantage of this method over the first method [for evanescent excitation] is that it is simpler- there is no need for a prism or wedge shaped surface and that there are no special requirements for the angle of incidence and shape of the excitation spot and one can use a simple CCD camera for imaging the fluorescence- and enables substantially smaller excitation volumes.

The device according to the present invention may comprise a surface hybridization zone of which an inner surface is coated with capture probes for multiple target nucleic acid sequences and a surface-specific detection system that detects those labels which are bound to the surface but does essentially not detect labels which are in solution.

Multiple different capture probes can be coated on the hybridization surface in a patterned array to simultaneously monitor the amplification of multiple different amplicons in the same surface hybridization zone. All capture spots monitor the amplification of a different amplicon within the same PCR mixture. This allows for much higher multiplex grades than currently possible. This allows for multiplex grades greater than 6 and up to 100 or more. An additional advantage of the embodiments of the invention is that only one fluorophore (one species) is needed which makes multiple expensive color filters and/or separated photodetectors unnecessary.

Thus, a device according to the present invention may be a micro-array.

In another embodiment of the invention the capture probes on the solid surface of the surface hybridization zone are folded probes (e.g. molecular beacons) or other probes (e.g. TaqMan probes) with a fluorescent label and quencher in close proximity to one another due to the structure of the probe. In this embodiment the PCR reaction(s) is (are) performed without any label in the reaction or label attached to the amplification primers. Specific amplicons that are formed during the PCR reaction(s) can hybridize with the labeled capture probes on the solid surface, thereby either separating quencher and label (in case of molecular beacon type folded probes) or allowing the polymerase to hydrolyze the probe (in case of TaqMan-like capture probes). As a result a fluorescent signal can be measured at the spot where the capture probes are located.

In this embodiment it is not a prerequisite to use a highly surface specific reader, since signal is only generated upon hybridization of amplicons to the capture probe. All other statements above also apply to this embodiment of the invention.

Thus, in some embodiments the capture probes are probes with a fluorescent label and a quencher in close proximity to one another due to the structure of the probes, and a signal may be detected in case an amplicon hybridizes to said capture probes.

In some embodiments, the surface hybridization zone of the device according to the present invention comprises an inner surface that is coated with capture probes for multiple target nucleic acid sequences, whereas in the capture probes are labeled and a signal may be detected in case an amplicon hybridizes to said capture probes.

According to this embodiment of the device the label is preferentially selected from a group comprising molecular beacons, intercalating dyes, TaqMan probes and the like.

In yet another embodiment of the device of the present invention the capture probes are immobilized on individually identifiable beads. In a preferred embodiment different beads have different capture probes.

It may be preferred that the beads are brought to the surface of the surface hybridization zone and captured amplicons are measured by surface-specific detection. The beads may be brought to the surface e.g. by magnetic actuation.

In a preferred embodiment the fluorescence of a label is detected. As outlined above, a person skilled in the art knows further fluorescent and non-fluorescent labels.

If the capture probes are immobilized on beads, this allows for a quasi-homogeneous assay as the beads are dispersed in the reaction solution. Quasi-homogeneous assays allow for faster kinetics than non-homogeneous assays. The beads can have a diameter between 50 nm and 3 $\mu$m and are individually identifiable e.g. by color-coding, bar-coding or size. Multiple beads containing different capture probes may be present in the same reaction solution. Beads with captured amplicons on them, can be brought to the surface by magnetic actuation (when (para)magnetic beads are used) or by dielectrophoresis (when non-magnetic beads are used). On the surface the beads can be identified and the captured amplicons can be detected by a surface-specific optical measurement.

Another method to distinguish different beads is based on the differences in resonance wavelength of the resonator modes propagating along the perimeter of the bead due to size differences. In this case the bead acts as a resonator that supports resonator modes propagating along the perimeter of the bead (for a spherical bead these resonator modes are so-called whispering gallery modes). The resonator is in-resonance for a wavelength where the phase shift after one roundtrip along the perimeter is a multiple of 2*pi. These resonator modes also have an evanescent field that extends into the environment (fluid) of the bead. A fluorophore in the near field region (typically <100 nm) of the bead can couple a fraction of its fluorescence to the resonator modes supported by the bead. The fraction coupled to the resonator mode is stronger for on-resonance wavelengths than for other wavelengths and this results in a modulation of the fluorescence spectrum with the resonance peaks corresponding to the resonator modes or whispering gallery modes. The typical diameter of the beads is larger than 1 $\mu$m up to 50 $\mu$m. Detection can be performed throughout the whole volume, because the fluorescence due to fluorophores that are not bound to the bead have the intrinsic spectrum of the fluorophore, and there is no need for a surface-specific optical measurement.

Thus, in a particular embodiment the surface hybridization zone of a device according to the present invention comprises individually identifiable beads wherein capture probes for multiple target nucleic acid sequences are immobilized on said individually identifiable beads.

In a preferred embodiment the device further comprises means for bringing the beads to the surface of the surface hybridization zone and a surface-specific detector that detects those labels which are bound to the surface but does essentially not detect labels which are in solution.

The methods and devices of the present invention are characterized in that hybridization and detection are performed in a designated surface hybridization zone. This has the advantage that the conditions for hybridization and detection can be adjusted independently from the amplification process. The thermal requirements of e.g. a PCR process and surface hybridization are very different. The PCR amplification process requires (fast) temperature cycling wherein part of the cycle the sample is above the dsDNA melting temperature. On the other hand the surface hybridization needs to take place at a temperature below the nucleic acid melting point, and for optimum performances diffusion limited local depletion of amplicons above the capture probe cites should be avoided.

The present multiple-zone concept allows decoupled optimization of these two processes, the volume amplification and the surface hybridization. Additionally, the detection is very accurate due to the relatively long time available for hybridization in each cycle. Some embodiments of the present invention provide a highly flexible method, as hybridization and detection can e.g. be performed from a certain number of cycle on or every $N^{th}$ circle.

The embodiments comprising no thermocycler zone and thus no rapid temperature changes have the additional advantage that they have a reduced risk of damage to the reaction container, cartridge and/or device due to a reduced temperature induced strain.

The methods and devices of the present invention allow for very sensitive detection of amplified target nucleic acids during and/or after amplification reactions.

The following examples are illustrative examples and are not limiting the scope of the invention.

EXAMPLES

Example 1: Assay optimization during real-time PCR using melting for accurate measurement of binding curves.

A straightforward measurement is to determine the number of PCR cycles for which the amplicon concentration of a certain target DNA is above threshold. In the ideal case (optimum PCR efficiency, no other sources of (statistical) errors) this enables the calculation of the initial copy number for the threshold PCR cycle number CT within the worst case a factor two difference between the estimated and the actual copy number (semiquantitative PCR). To improve the accuracy rather the hybridization curve is measured, that is the amount of hybridized DNA per unit area vs. the hybridization time, preferably already from the start of the hybridization because then the surface concentration of hybridized DNA is sufficiently far away from equilibrium. The hybridization zones are monitored for multiple PCR cycles.

Individual temperature zones can be reconfigured as follows:

1. Determine CT
2. Perform melting step (high temperature) for a hybridization zone after at least CT-1 PCR cycles -> no hybridized amplicons
3. Reduce temperature to hybridization temperature and start measuring the hybridization curve for a capture probe specific for the target DNA.
4. Determine accurate estimate for amplicon concentration .
5. Determine contribution of aspecific binding by making a melting curve.
6. Correct for contribution of aspecific binding -> accurate and correct estimate for amplicon concentration
7. Determine initial copy number.

Example 2: Assay optimization during real-time PCR using split temperature zones for hybridization into two subzones with only one subzone (A) overlapping with the hybridization zone.

If hybridization is desired only after N cycles, because the amplicon concentration is simply too low to detect during cycles < N-1, then the temperature of the surface hybridization zones for the PCR cycles until cycle N-1 ("subzones A") is set sufficiently above the hybridization temperature (e.g. the denaturation temperature) in order to avoid hybridization. In the surface hybridization zones for the PCR cycles starting from cycle N ("subzones B") the temperature is set to the annealing temperature for primer annealing.

If the size of the heater elements can be made sufficiently small and the cross talk between subsequent cells (e.g., due to convection) is sufficiently small, it is also possible to split the hybridization zone into subzones where in each subzone are capture probes having a similar optimal hybridization temperature. This way the hybridization of each target is performed under the optimal temperature conditions.

Example 3: Detection methods for real-time PCR

Real-time array PCR (with in total $N_{max}$ cycles) can be performed by directing the PCR fluid through the meandering structure, wherein the time for the different temperature steps can be controlled by the flow rate (pressure) and the layout of the meander, and detection of the hybridized amplicons. Detection may be performed in different ways, these are, inter alia:

Detection of the hybridization during each cycle in the time that hybridization takes place, which enables to determine the binding kinetics or slope of the measured fluorescent signal for a hybridization spot as a function of time. The slope correlates to the concentration of target molecules (amplicons). This approach requires relatively fast scanning/measurements over multiple hybridization zones.

Detection after the $N_{max}$th cycle, which results in equilibrium values (frozen picture) for the measured fluorescent signal. This approach considerably relaxes the requirements for the scanning/measurement speed.

A scheme in which the scanning procedure is adapted to the measured hybridization signals, e.g. by first performing a detection at an hybridization zone corresponding to a large number of cycles to determine which hybridization spots give a signal at all. In a next step the hybridization zone for a cycle where the hybridization signal is not in equilibrium is chosen to determine the slope of the hybridization signal vs. time curve, which results in a quantitative estimate for the concentration. For a detection limit of 100 pM, for an association constant $k_{on}$=10^5 M^{-1}s^{-1} and a dissociation constant $k_{off}$=10^{-6} s^{-1}, this corresponds to a hybridization time (corresponding to a surface concentration of hybridized amplicons corresponding to 63% of the equilibrium concentration) of $\tau$=90000 s, for a concentration of 10 nM $\tau$=1000 s. Assuming that each PCR cycle takes 90 s and the PCR efficiency is 100 %, there are two orders of magnitude difference to the concentration corresponding to 7 cycles (630 s). After 1000+630=1630 s the hybridization signal for a concentration of 100 pM is still not in equilibrium and one can determine the concentration by measuring the slope of the hybridization curve.

One can also think of washing away afterwards the PCR buffer. This results in frozen pictures of spots with hybridized amplicons for a give cycle number and a given hybridization time. This method has the disadvantage that one cannot determine the slope of the hybridization vs. time curve (less accurate estimate for the amplicon concentrations), but has the advantage that the washing removes the labels in the PCR buffer and makes the measurement less sensitive for the fluorescent background. An additional advantage is that after the fluid has passed the hybridization spots and hybridization has been measured onto the array, washing fluid may pass the spots (or a different kind of buffer like hybridization buffer). Subsequently the temperature of this zone can be increased up to 95 °C and melting temperatures can be measured for every individual spot.

**Claims**

1. Method for amplification and detection of target nucleic acid sequences in an amplification solution in a reaction container, comprising the steps of
providing a reaction container comprising at least one surface hybridization zone in which capture probes are immobilized on a surface, wherein said capture probes are substantially complementary to regions on said target nucleic acid sequences;
adding the amplification solution to said reaction container;
generating a temperature zone profile in the reaction container with at least two kinds of thermally decoupled zones, wherein one kind of zone is identical or at least overlapping to/with the surface hybridization zones, wherein the surface hybridization zones have a generated temperature allowing for hybridization of the capture probes to the target nucleic acid sequences;
performing an amplification of target nucleic acid sequences in the reaction container; and
detecting amplified nucleic acid sequences in periodic or defined intervals during and/or after amplification, wherein amplified nucleic acid sequences are detected by hybridization of capture probes to said amplified nucleic acid sequences in said surface hybridization zone.

2. Method according to claim 1,
wherein said amplification solution is passed through said at least two thermally decoupled zones during amplification and detection.

3. Method according to claims 1 to 2, wherein at least one surface hybridization zone is used for hybridization and detection of amplified target nucleic acid sequences at multiple stages of the entire amplification process.

4. Method according to claim 3,
wherein at least one hybridization zone is used for hybridization and detection at multiple stages of the entire amplification process, and
wherein two or more of the at least two thermally decoupled zones are comprised in the same compartment or volume of the reaction container.

5. Method according to claim 3,
wherein at least one hybridization zone is used for hybridization and detection at multiple stages of the entire amplification process, and
wherein the at least two thermally decoupled zones are comprised in separate compartments or volumes of the reaction container.

6. Method according to claim 4 or 5,
wherein the reaction container comprises at least one surface hybridization zone with substantially constant generated temperature and at least one thermocycler zone with variable temperature in the range of from the melting point to the boiling point of the amplification solution, wherein the amplification solution is transferred from the thermocycler zone to the surface hybridization zone for detection of the amplified target nucleic acid and vice versa for further amplification.

7. Method according to claim 6, wherein the reaction container comprises at least one thermocycler zone and at least one surface hybridization zone, wherein the amplification reaction is a polymerase chain reaction (PCR) and wherein at least denaturation and primer extension of the polymerase chain reaction are performed in the thermocycler zone and the temperature in the thermocycler zone is cycled at least between denaturation temperature and extension temperature.

8. Method according to claim 7, wherein the primer annealing to said target nucleic acid is performed in the thermocycler zone and wherein the temperature in the thermocycler zone is cycled between denaturation temperature, annealing temperature and extension temperature.

9. Method according to claim 6 or 7, wherein the temperature in the surface hybridization zone is suitable for primer annealing to said target nucleic acid and wherein primer annealing to said target nucleic acid is performed in the surface hybridization zone.

10. Method according to claims 1 or 2,
wherein each surface hybridization zone is used for hybridization and detection of amplified target nucleic acid sequences only at a particular stage of the entire amplification process, and
wherein in the reaction container two or more kinds of thermally decoupled zones are generated,
wherein each zone has a substantially constant generated temperature, and
wherein the first kind of zone is a surface hybridization zone and the second and further kind is an amplification zone,
wherein the amplification solution is passed through all zones such that for each amplification cycle at least one amplification zone is passed through and for each amplification cycle in which hybridization and detection is desired additionally a surface hybridization zone is passed through,
and wherein the transport is unidirectional and non-circular.

11. Method according to claim 10,
wherein the temperature in all zones of one kind is equal and adjusted concertedly or the temperature in all zones is adjusted separately.

12. Method according to any one of the claims 1 to 11, wherein multiple nucleic acid sequences are detected by at least one capture probe complementary to each target nucleic acid sequence to be detected.

13. A device for amplification and detection of target nucleic acid sequences in an amplification solution in a reaction container, comprising
a reaction container for receiving an amplification solution comprising said target nucleic acid sequences, wherein the reaction container comprises at least one surface hybridization zone in which capture probes are immobilized on a surface, wherein said capture probes are substantially complementary to regions on said target nucleic acid sequences and at least one other kind of zone;
one or more temperature controllers for controlling a temperature profile of at least two kind of temperature zones in said reaction container, wherein one kind of zone is substantially overlapping with said surface hybridization zones, and wherein the surface hybridization zone has a substantially constant generated temperature allowing for hybridization of capture probes to complementary target nucleic acid sequences;
a detection system that detects target nucleic acid sequences which are bound to said capture probes but does essentially not detect target nucleic acid sequences which are not bound to said capture probes; and
a transportation system for transporting the amplification solution between the zones.

14. Device according to claim 13, wherein said reaction container is comprised in an exchangeable cartridge.

15. Device according to any one of the claims 13 or 14 for amplification and detection of target nucleic acid sequences in a polymerase chain reaction (PCR).

16. Device according to any one of the claims 13 to 15, wherein at least one surface hybridization zone can be used for hybridization and detection of amplified target nucleic acid sequences at more than one stages of the entire amplification process.

17. Device according to any one of the claims 16, wherein two or more of the at least two temperature zones are comprised either in the same compartment or volume of the reaction container or alternatively wherein the at least two temperature zones are comprised in separate compartments or volumes of the reaction container.

18. Device according to any one of the claims 16 to 17 comprising at least one surface hybridization zone and at least one thermocycler zone of which the temperature can be cycled in the range of from the melting point to the boiling point of the amplification solution.

19. Device according to any one of the claims 13 to 18, wherein the reaction container comprises at least one surface hybridization zone, at least one generated denaturation zone and at least one generated extension zone.

20. Device according to claims 13 to 19, wherein a plurality of surface hybridization zones is present in the reaction container such that at two or more stages of the entire amplification process surface hybridization may occur.

21. Device according to any one of the claims 13 to 20, wherein the reaction container comprises three kinds of thermally decoupled zones, wherein the generated temperature in each zone may be kept substantially constant, and wherein the first kind of zone is an annealing zone, the second kind is a denaturation zone and the third kind is an extension zone, and wherein for each amplification cycle for which detection is desired one surface hybridization zone is present or the annealing zone is substantially overlapping with the surface hybridization zone, wherein the amplification solution is passed through all zones such that for each amplification cycle first a denaturation zone, secondly an annealing zone and thirdly an extension zone is passed, wherein the transport is unidirectional and non-circular, and wherein the denaturation zones have a generated temperature allowing for denaturation of the target nucleic acid sequences, the annealing zones have a temperature allowing for annealing of primers, the surface hybridization zones have a generated temperature allowing for annealing of primers and hybridization of a capture probe and the extension zones have a generated temperature allowing for primer extension.

22. A cartridge for amplification and detection of target nucleic acid sequences in an amplification solution in a reaction container, comprising a reaction container for receiving an amplification solution comprising said target nucleic acid sequences, wherein the reaction container comprises at least one surface hybridization zone in which capture probes are immobilized on a surface, wherein said capture probes are substantially complementary to regions on said target nucleic acid sequences and at least one other kind of zone.

23. A device for receiving the cartridge of claim 22, comprising:

one or more temperature controllers and/or temperature adjusters for generating a temperature profile of at least two kind of temperature zones in a reaction container comprised in said cartridge, wherein one kind of zone has a substantially constant generated temperature allowing for hybridization of capture probes to complementary target nucleic acid sequences and wherein the temperature controllers and/or temperature adjusters control, adjust and maintain the temperature in the zones;
a detection system that detects targets which are bound to said capture probes but does essentially not detect targets which are not bound to said capture probes;
a transportation system for transporting the amplification solution between the zones; and
a receiving element for said cartridge.

24. Use of a method according to claims 1 to 12 or a device according to claims 13 to 21 or 23 or the cartridge according to claim 22 for quantitative analysis of target nucleic acid sequences, for simultaneous quantitative analysis of multiple target nucleic acid sequences or for analyzing a sample for the presence of a target nucleic acid.

25. Use according to claim 24 for clinical diagnosis, point-of care diagnosis, bio-molecular diagnostics, gene or protein expression arrays, environmental sensors, food quality sensors or forensic applications.

26. Use of a method according to claims 1 to 12 or a device according to claims 13 to 21 or 23 or the cartridge according to claim 22 in real-time PCR or real-time multiplex PCR.

Fig 1:

Real-time PCR 6-plex in 1 chamber

denaturation    annealing    elongation

hybridization detection

Real-time Array PCR

Fig 2:

Thermal cycling    Constant Temp

PCR Amplification Zone    Surface Hybridization Zone

Unique Sample Volume

Fig 3:

Thermal cycling      Constant Temp

PCR Amplification Zone

Surface Hybridization Zone

Split Sample Volume

Fig 4:

Thermal cycling      Constant Temp

PCR Amplification Zone

Surface Hybridization Zone

Split Sample Volume

Fig 5:

Surface Hybridization Zone

Denaturation Zone ~ 95°C → Annealing Zone ~ 54°C → Extension Zone ~ 72°C

Split Sample Volume
Over three constant temperature zones

Fig 6:

Surface Hybridization Zone

Denaturation Zone ~ 95°C → Annealing Zone ~ 54°C

Split Sample Volume
Over two constant temperature zones

Fig 7:

Capture probes

Fig 8:

Fig 11:

(20)

(21)

(1)   (2)   (3)

(11)

N          N+1          N+2

Fig 12:

N   N+1   N+2

(1)

(20)

(3)

(2)

(21)

(1)

(10)

(3)

Fig 13:

Fig 14:

(51)                         (42)

A                        A

(20)

(40)        (41)

(51)     (41)

(20-I)

50

Fig 15:

100

Fig 16:

Europäisches Patentamt

European Patent Office

Office européen des brevets

## EUROPEAN SEARCH REPORT

Application Number

EP 08 15 8774

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 788 097 A (EPPENDORF ARRAY TECHNOLOGIES S [BE]) 23 May 2007 (2007-05-23) see whole doc, esp. claims, Figs, 6 and 7 and page 5 f.. | 1-26 | INV. C12Q1/68 |
| X | EP 1 788 095 A (EPPENDORF ARRAY TECHNOLOGIES S [BE]) 23 May 2007 (2007-05-23) see whole doc. esp. claims and Figs. | 1-26 | |
| X | WO 2008/047272 A (KONINKL PHILIPS ELECTRONICS NV [NL]; VOSSENAAR ERIK R [NL]; KLUNDER DE) 24 April 2008 (2008-04-24) see whole doc. esp. claims | 1-26 | |

TECHNICAL FIELDS
SEARCHED (IPC)

C12Q

The present search report has been drawn up for all claims

| Place of search | Date of completion of the search | Examiner |
|---|---|---|
| Munich | 7 January 2009 | Mueller, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 08 15 8774

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2009

| Patent document cited in search report | | Publication date | Patent family member(s) | | Publication date |
|---|---|---|---|---|---|
| EP 1788097 | A | 23-05-2007 | NONE | | |
| EP 1788095 | A | 23-05-2007 | EP | 1788096 A1 | 23-05-2007 |
|  | | | EP | 1788098 A1 | 23-05-2007 |
| WO 2008047272 | A | 24-04-2008 | NONE | | |

EPO FORM P0459

For more details about this annex : see Official Journal of the European Patent Office, No. 12/82

## REFERENCES CITED IN THE DESCRIPTION

**Patent documents cited in the description**

- WO 9727317 A
- WO 0072018 A
- WO 0131055 A
- US 5744305 A
- US 6346413 B
- WO 9727329 A